# EUROPÄISCHE PATENTSCHRIFT

(11) **EP 1 345 575 B1**
(45) Veröffentlichungstag und Bekanntmachung des Hinweises auf die Patenterteilung: **20.01.2010**
(21) Anmeldenummer: 01989524.2
(22) Anmeldetag: 28.11.2001
(51) Int. Cl.: A61K 8/81, A61K 8/895, A61K 8/894, A61K 8/86, A61K 8/91, A61Q 5/00, A61Q 19/00

(54) **KOSMETISCHE UND DERMATOLOGISCHE HAARBEHANDLUNGSMITTEL**
COSMETIC AND DERMATOLOGICAL HAIR-TREATMENT AGENTS
PRODUITS CAPILLAIRES COSMETIQUES ET DERMATOLOGIQUES

(30) Priorität: 01.12.2000 DE 10059827
(43) Veröffentlichungstag der Anmeldung: 24.09.2003
(73) Patentinhaber: Clariant Produkte (Deutschland) GmbH, 65929 Frankfurt am Main (DE)
(72) Erfinder: LÖFFLER, Matthias, 65527 Niedernhausen (DE); MORSCHHÄUSER, Roman, 55122 Mainz (DE); GLAUDER, Jan, 65812 Bad Soden (DE)
(86) Internationale Anmeldenummer: PCT/EP2001/013862
(87) Internationale Veröffentlichungsnummer: WO 2002/043677

(56) Entgegenhaltungen:
- EP-A- 0 503 853
- DE-A- 19 907 715
- US-A- 4 713 236
- US-A- 4 859 458
- US-A- 5 275 809

## Beschreibung

Die vorliegende Erfindung betrifft kosmetische und dermatologische Haarbehandlungsmittel, enthaltend kammförmige Copolymere auf Basis von Acryloyldimethyltaurinsäure.

Durch oftmaliges Bleichen, Dauerwellen und Färben, aber auch durch häufiges Waschen der Haare mit entfettenden Tensiden kommt es zu einer Schädigung der Haarstruktur. Das Haar wird spröde und verliert seinen Glanz. Angeraute Haaroberflächen verursachen Verfilzungen sowie Verknotungen der Haare, die Kämmbarkeit wird erschwert. Infolgedessen haben Haarbehandlungsmittel, die die Glanz, Nass- und Trockenkämmbarkeit, Konditionierung, und Farbtiefe der Haare verbessern, eine erhebliche Bedeutung erlangt. Darüber hinaus sollen Haarpflegemittel durch kürzere Trocknungszeiten die Hitzebelastung der Haare beim Fönen verringern und bereits vorhandene Haarschädigungen, wie z.B. Splisse, "reparieren". Derartige Mittel werden beispielsweise häufig in Form einer klaren Haarpflegespülung, eines Aerosolschaumes oder auch in Emulsionsform als sogenannte Creme-Rinses nach der Haarwäsche im noch nassen Haar verteilt und je nach Art des Haarbehandlungsmittels entweder nach einigen Minuten Einwirkzeit mit Wasser ausgespült oder aber auf dem Haar belassen.

Die Patentliteratur enthält zahlreiche Vorschläge zur Realisierung eines solchen Vorhabens, darunter die Verwendung von wasserlöslichen Polymeren, kationischen Fettsäurederivaten, hauptsächlich kationischen, insbesondere quarternären Ammoniumverbindungen, wie Cetyltrimethylammoniumchlorid, alleine oder in Kombination mit verschiedenen wachsartigen Zusätzen, wie zum Beispiel Kohlenwasserstoffen, Fettalkoholen und Fettsäuren. Auch Öle und ölähnliche Substanzen, wie beispielsweise flüssige Kohlenwasserstoffverbindungen, Fettalkohole, Monocarboxysäureester (monocarboxylic acid ester), Polyalkoholester, wasserlösliche Silikonen und Emulsionen von Silikonen und anderen Ölen werden beschrieben.
Ein Nachteil der oben beschriebenen Mittel besteht darin, dass diese nach dem Abspülen oftmals den feuchten Haaren einen klebrigen Griff verleihen und die trockenen Haare beschweren.
Häufig benutzte Materialien, die das Haar geschmeidig und weich erscheinen lassen, sind Silikonderivate. Silikone haben jedoch den Nachteil, insbesondere dünnen Haare so weich zu machen, dass diese kaum noch frisierbar sind.
Zudem lassen sich unlösliche Silikone, wie sie z.B. in US 2,826,551 vorgeschlagen werden, oft nur unzureichend in Formulierungen einarbeiten. Das Problem besteht hier in der Erzeugung einer Suspension der feinverteilten, unlöslichen Polymere, die über einen längeren Zeitraum stabil sein soll. Eine Vielzahl von Verbindungen wurden den silikonhaltigen Formulierungen zugesetzt, um eine Verdickung und Stabilisierung zu bewirken. Die bisher erfolgreichste Vorgehensweise wird in EP 0,181,773 beschrieben, wo die Verwendung langkettiger Acylderivate zur Bildung stabiler Formulierungen führt. Die Acylderivate enthalten Fettsäurealkanolamide, Fettsäuredialkanolamide, Alkanolamide und deren Derivate. Diese Amide stehen im Verdacht, an der Bildung von Nitrosaminen beteiligt zu sein. Es ist daher erwünscht, kosmetische Zubereitungen ohne solche Derivate zu formulieren.
Haarpflegemittel müssen zudem auch eine Viskosität haben, die dem jeweiligen Verwendungszweck angepasst ist und sich möglichst variabel einstellen lässt. So werden z.B. von einem Haargel oder einer Haarkurcreme relativ hohe Viskositäten gefordert, während eine Haarspülung gewöhnlich eine fließfähige Flüssigkeit mit einer relativ niedrigen Viskosität darstellt.
Als Verdicker und Gelbildner sind insbesondere die auf der Basis der Poly(meth)acrylsäure hergestellten Polyacrylsäuren und deren wasserlösliche Copolymere bekannt. Die Vielfalt der möglichen Strukturen und die damit verbundenen vielfältigen Anwendungsmöglichkeiten drücken sich nicht zuletzt in einer Vielzahl von neuen Patenten aus, die seit Mitte der 70iger Jahre weltweit angemeldet wurden. Ein wesentlicher Nachteil dieser Verdicker auf Basis von Poly(meth)acrylsäure ist die starke pH-Abhängigkeit der Verdickungsleistung. So wird im allgemeinen Viskosität nur dann aufgebaut, wenn der pH Wert der Formulierung oberhalb von pH 6 eingestellt ist und somit die Poly(meth)acrylsäure in neutralisierter Form vorliegt. Ferner sind die entsprechenden Gele/Formulierung empfindlich gegenüber UV-Strahlung und Scherung und vermitteln auf der Haut und auf den Haaren zudem ein klebriges Gefühl. Auch die Handhabung dieser Verdickerpolymere ist verbesserungswürdig. Da die Verdicker auf Basis von Poly(meth)acrylsäure i.a. in saurer Form vorliegen, bedarf es z.B. bei der Formulierung eines zusätzlichen Neutralisationsschrittes.
In den 90iger Jahren wurden neuartige Verdicker auf Basis vernetzter und neutralisierter Acryloyldimethyltaurate in den Markt eingeführt (EP-B-0 815 828, EP-B-0 815 844, EP-B-0 815 845 und EP-B-0 829 258). Sowohl in Form des vorneutralisierten Homopolymers als auch als korrespondierendes Copolymer (^{®}Aristoflex AVC, Clariant GmbH) zeigen sich diese auf Sulfonatgruppen basierenden Typen den Poly(meth)acrylaten in vieler Hinsicht überlegen. Beispielsweise zeigen Acryloyldimethyltaurat-basierende Verdickersysteme hervorragende Eigenschaften in pH-Bereichen unterhalb von pH 6, also in einem pH-Bereich, in dem mit herkömmlichen Poly(meth)acrylat-Verdickern nicht mehr gearbeitet werden kann. Hohe UV- und Scherstabilität, hervorragende viskoelastische Eigenschaften, leichte Verarbeitbarkeit und ein günstiges toxikologische Profil des Hauptmonomeren machen Acryloyldimethyltauratbasierende Verdickersysteme zu modernen, neuen Vertretern mit hohem Potenzial für die Zukunft.
Im Laufe der letzten Jahre etablierte sich ein weiteres Verdickerkonzept auf dem Markt. Hierbei wurde durch hydrophobe Modifikation der konventionellen Poly(meth)acrylate der Zugang zu Polymeren gefunden, die sowohl verdickende als auch emulgierende/dispergierende Eigenschaften aufweisen können. Beispiele für kommerzielle hydrophob modifizierte Poly(meth)acrylate sind ^{®}Pemulen TR-1 und TR-2 von BF-Goodrich und ^{®}Aculyn 22 von Rohm und Haas. Da diese hydrophob modifizierte Polymere ausnahmslos auf der Basis von (Meth)acrylsäure aufgebaut sind, besitzen sie auch die oben erwähnten Nachteile der Poly(meth)acrylate.

US 4 859 458 offenbart Zubereitungen zur Haar Konditionierung, die Copolymere aus AMPS und neutralen, anionischen oder Kationischen Monomeren enthalten.

Überraschend wurde nun gefunden, dass sich eine neue Klasse von Kammpolymeren auf Basis von Acryloyldimethyltaurinsäure (AMPS) - die sich sowohl als Konditionierer, Glättmittel, Antistatikmittel, Konsistenzgeber, Emulgator, Dispergator, Gleitmittel und Stabilisator eignet- hervorragend zur Formulierung von kosmetischen und dermatologischen Haarbehandlungsmitteln eignet.

Gegenstand der Erfindung sind kosmetische und dermatologische Haarbehandlungsmittel, enthaltend mindestens ein Copolymer, erhältlich durch radikalische Copolymerisation von
A) Acryloyldimethyltaurinsäure und/oder Acryloyldimethyltauraten,
B) gegebenenfalls einem oder mehreren weiteren olefinisch ungesättigten, nicht kationischen, gegebenenfalls vernetzenden, Comonomeren, die wenigstens ein Sauerstoff-, Stickstoff-, Schwefel- oder Phosphoratom aufweisen und ein Molekulargewicht kleiner 500 g/mol besitzen,
C) einem oder mehreren olefinisch ungesättigten, kationischen Comonomeren, die wenigstens ein Sauerstoff-, Stickstoff-, Schwefel- oder Phosphoratom aufweisen und ein Molekulargewicht kleiner 500 g/mol besitzen,
D) gegebenenfalls einer oder mehreren mindestens monofunktionellen zur radikalischen Polymerisation befähigten, siliziumhaltigen Komponente(n),
E) gegebenenfalls einer oder mehreren mindestens monofunktionellen, zur radikalischen Polymerisation befähigten, fluorhaltigen Komponente(n),
F) einem oder mehreren einfach oder mehrfach olefinisch ungesättigten, gegebenenfalls vernetzenden, Makromonomeren, die jeweils mindestens ein Sauerstoff-, Stickstoff-, Schwefel- oder Phosphoratom besitzen und ein zahlenmittleres Molekulargewicht größer oder gleich 200 g/mol aufweisen, wobei es sich bei den Makromonomeren nicht um eine siliziumhaltige Komponente D) oder fluorhaltige Komponente E) handelt, und wobei es
   sich bei den Makromonomeren F) um Verbindungen der Formel (IV) handelt, 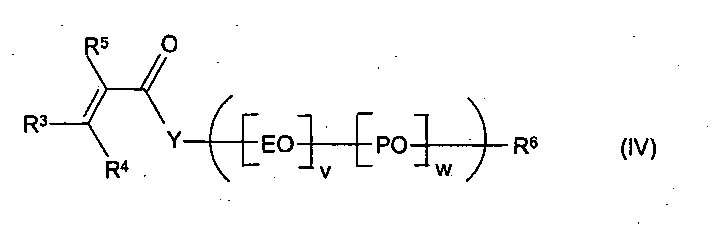 worin
   R₃ und R₄ gleich H oder -CH₃ sind,
   R₅ gleich H oder -CH₃ ist;
   R₆ gleich einem n-aliphatischen, iso-aliphatischen, olefinischen, cycloaliphatischen, arylaliphatischen oder aromatischen (C₁-C₃₀)-Kohlenwasserstoffrest ist,
   EO eine Ethylenoxideinheit und
   PO eine Propylenoxideinheit ist,
   v und w unabhängig voneinander 0 bis 500 betragen, wobei die Summe aus v und w im Mittel ≥ 1 sein muss und die Verteilung der EO- und PO-Einheiten über die Makromonomerkette statistisch, blockartig, alternierend oder gradientenartig sein kann und
   Y -O-, -C(O)-, -C(O)-O-, -S-, -O-CH₂-CH(O-)CH₂OH, -O-CH₂-CH(OH)-CH₂O-, -O-SO₂-O-, -O-SO-O-, -PH-, -P(CH₃)-, -PO₃-, -NH-oder -N(CH₃)- ist, und
G) wobei die Copolymerisation gegebenenfalls in Gegenwart mindestens eines polymeren Additivs mit zahlenmittleren Molekulargewichten von 200 g/mol bis 10⁹ g/mol erfolgt.

Die erfindungsgemäßen Copolymere besitzen bevorzugt ein Molekulargewicht von 10³ g/mol bis 10⁹ g/mol, besonders bevorzugt von 10⁴ bis 10⁷ g/mol, insbesondere bevorzugt 5*10⁴ bis 5*10⁶ g/mol.

Bei den Acryloyldimethyltauraten kann es sich um die anorganischen oder organischen Salze der Acryloyldimethyltaurinsäure (Acrylamidopropyl-2-methyl-2-sulfonsäure) handeln. Bevorzugt werden die Li⁺-, Na⁺-, K⁺-, Mg⁺⁺-, Ca⁺⁺-, Al⁺⁺⁺-und/oder NH₄⁺-Salze. Ebenfalls bevorzugt sind die Monoalkylammonium-, Dialkylammonium-, Trialkylammonium- und/oder Tetraalkylammoniumsalze, wobei es sich bei den Alkylsubstituenten der Amine unabhängig voneinander um (C₁-C₂₂)-Alkylreste oder (C₂-C₁₀)-Hydroxyalkylreste handeln kann. Weiterhin sind auch ein bis dreifach ethoxylierte Ammoniumverbindungen mit unterschiedlichem Ethoxylierungsgrad bevorzugt. Es sollte angemerkt werden, dass auch Mischungen von zwei- oder mehreren der oben genannten Vertreter im Sinne der Erfindung sind.

Der Neutralisationsgrad der Acryloyldimethyltaurinsäure kann zwischen 0 und 100 % betragen, besonders bevorzugt ist ein Neutralisationsgrad von oberhalb 80%.

Bezogen auf die Gesamtmasse der Copolymere beträgt der Gehalt an Acryloyldimethyltaurinsäure bzw. Acryloyldimethyltauraten mindestens 0,1 Gew.-%, bevorzugt 20 bis 99,5 Gew.-%, besonders bevorzugt 50 bis 98 Gew.-% betragen.

Als Comonomere B) können alle olefinisch ungesättigten, nicht kationischen Monomere eingesetzt werden, deren Reaktionsparameter eine Copolymerisation mit Acryloyldimethyltaurinsäure und/oder Acryloyldimethyltauraten in den jeweiligen Reaktionsmedien erlauben.

Als Comonomere B) bevorzugt sind ungesättigte Carbonsäuren und deren Anhydride und Salze, sowie deren Ester mit aliphatischen, olefinischen, cycloaliphatischen, arylaliphatischen oder aromatischen Alkoholen mit einer Kohlenstoffzahl von 1 bis 30.
Als ungesättigte Carbonsäuren besonders bevorzugt sind Acrylsäure, Methacrylsäure, Styrolsulfonsäure, Maleinsäure, Fumarsäure, Crotonsäure, Itaconsäure und Seneciosäure.
Als Gegenionen bevorzugt sind Li⁺, Na⁺, K⁺, Mg⁺⁺, -Ca⁺⁺, Al⁺⁺⁺, NH₄⁺, Monoalkylammonium-, Dialkylammonium-, Trialkylammonium- und/oder Tetraalkylammoniumreste, wobei es sich bei den Alkylsubstituenten der Amine unabhängig voneinander um (C₁-C₂₂)-Alkylreste oder (C₂-C₁₀)-Hydroxyalkylreste handeln kann. Zusätzlich können auch ein bis dreifach ethoxylierte Ammoniumverbindungen mit unterschiedlichem Ethoxylierungsgrad Anwendung finden. Der Neutralisationsgrad der Carbonsäuren kann zwischen 0 und 100% betragen.
Als Comonomere B) weiterhin bevorzugt sind offenkettige N-Vinylamide, bevorzugt N-Vinylformamid (VIFA), N-Vinylmethylformamid, N-Vinylmethylacetamid (\/IMA) und N-Vinylacetamid; cyclische N-Vinylamide (N-Vinyllactame) mit einer Ringgröße von 3 bis 9, bevorzugt N-Vinylpyrrolidon (NVP) und N-Vinylcaprolactam; Amide der Acryl- und Methacrylsäure, bevorzugt Acrylamid, Methacrylamid, N,N-Dimethylacrylamid, N,N-Diethylacrylamid und N,N-Diisopropylacrylamid; alkoxylierte Acryl-und Methacrylamide, bevorzugt Hydroxyethylmethacrylat, Hydroxymethylmethacrylamid, Hydroxyethylmethacrylamid, Hydroxypropylmethacrylamid und Bernsteinsäuremono-[2-(methacryloyloxy)-ethylester]; N,N-Dimethylaminomethacrylat; Diethylamino-methylmethacrylat; Acryl- und Methacrylamidoglykolsäure; 2- und 4-Vinylpyridin; Vinylacetat; Methacrylsäureglycidylester; Styrol; Acrylnitril; Vinylchlorid; Stearylacrylat; Laurylmethacrylat; Vinylidenchlorid; und/oder Tetrafluorethylen.
Als Comonomere B) ebenfalls geeignet sind anorganische Säuren und deren Salze und Ester. Bevorzugte Säuren sind Vinylphosphonsäure, Vinylsulfonsäure, Allylphosphonsäure und Methallylsulfonsäure.

Der Gewichtsanteil der Comonomere B), bezogen auf die Gesamtmasse der Copolymere, kann 0 bis 99,8 Gew.-% betragen und beträgt bevorzugt 0,5 bis 80 Gew.%, besonders bevorzugt 2 bis 50 Gew.%.

Als Comonomere C) kommen alle olefinisch ungesättigten Monomere mit kationischer Ladung in Frage, die in der Lage sind, in den gewählten Reaktionsmedien mit Acryloyldimethyltaurinsäure oder deren Salze Copolymere zu bilden. Die dabei resultierende Verteilung der kationischen Ladungen über die Ketten hinweg kann statistisch, alternierend, block- oder gradientenartig sein. Es sei darauf hingewiesen werden, dass unter den kationischen Comonomeren C) auch solche zu verstehen sind, die die kationische Ladung in Form einer betainischen, zwitterionischen, oder amphoteren Struktur tragen.
Comonomere C) im Sinne der Erfindung sind auch aminofunktionalisierte Precursor, die durch polymeranaloge Reaktionen in Ihre entsprechenden quaternären (z.B. Reaktion mit Dimethylsulfat, Methylchlorid), zwitterionischen (z.B. Reaktion mit Wasserstoffperoxid), betainischen (z.B. Reaktion mit Chloressigsäure), oder amphomeren Derivate überführt werden können.

Besonders bevorzugt als Comonomere C) sind
Diallyldimethylammoniumchlorid (DADMAC),
[2-(Methacryloyloxy)ethyl]trimethylammoniumchlorid (MAPTAC),
[2-(Acryloyloxy)ethyl]trimethylammoniumchlorid,
[2-Methacrylamidoethyl]trimethylammoniumchlorid,
[2-(Acrylamido)ethyl]trimethylammoniumchlorid,
N-Methyl-2-vinylpyridiniumchlorid
N-Methyl-4-vinylpyridiniumchlorid
Dimethylaminoethylmethacrylat,
Dimethylaminopropylmethacrylamid,
Methacryloylethyl-N-oxid und/oder
Methacryloylethyl-betain.

Der Gewichtsanteil der Comonomeren C) kann, bezogen auf die Gesamtmasse der Copolymere, 0,1 bis 99,8 Gew.-%, bevorzugt 0,5 bis 30 Gew.% und besonders bevorzugt 1 bis 20 Gew.-% betragen.

Als polymerisationsfähige, siliziumhaltige Komponenten D) sind alle mindestens einfach olefinisch ungesättigten Verbindungen geeignet, die unter den jeweils gewählten Reaktionsbedingungen zur radikalischen Copolymerisation befähigt sind. Dabei muss die Verteilung der einzelnen silikonhaltigen Monomere über die entstehenden Polymerketten hinweg nicht notwendigerweise statistisch erfolgen. Auch die Ausbildung von beispielsweise block- (auch multiblock-) oder gradientenartigen Strukturen ist im Sinne der Erfindung. Kombinationen von zwei oder mehreren unterschiedlichen silikonhaltigen Vertretern sind auch möglich. Die Verwendung von silikonhaltigen Komponenten mit zwei oder mehr polymerisationsaktiven Gruppen führt zum Aufbau verzweigter oder vernetzter Strukturen.

Bevorzugte silikonhaltige Komponenten sind solche gemäß Formel (I).

R¹ - Z- [(Si(R³R⁴)-O-)_{w}-(Si(R⁵R⁶)-O)ₓ-]- R² (I)

Dabei stellt R¹ eine polymerisationsfähige Funktion aus der Gruppe der vinylisch ungesättigten Verbindungen dar, die zum Aufbau polymerer Strukturen auf radikalischem Wege geeignet ist. Bevorzugt stellt R¹ einen Vinyl-, Allyl-, Methallyl-, Methylvinyl-, Acryl- (CH₂=CH-CO-), Methacryl- (CH₂=C[CH₃]-CO-), Crotonyl-, Senecionyl-, Itaconyl-, Maleinyl-, Fumaryl- oder Styrylrest dar.
Zur Anbindung der silikonhaltigen Polymerkette an die reaktive Endgruppe R¹ ist eine geeignete chemische Brücke Z erforderlich. Bevorzugte Brücken Z sind -O-, - ((C₁ - C₅₀)Alkylen)-, -((C₆-C₃₀) Arylen)-, -((C₅ - C₈) Cycloalkylen)-, -((C₁-C₅₀)Alkenylen)-, -(Polypropylenoxid)ₙ-, -(Polyethylenoxid)ₒ-, -(Polypropylenoxid)ₙ(Polyethylenoxid)ₒ-, wobei n und o unabhängig voneinander Zahlen von 0 bis 200 bedeuten und die Verteilung der EO/PO-Einheiten statistisch oder blockförmig sein kann. Weiterhin geeignet als Brückegruppierungen Z sind -((C₁ - C₁₀)Alkyl)-(Si(OCH₃)₂)- und -(Si(OCH₃)₂)-.
Der polymere Mittelteil wird durch silikonhaltige Wiederholungseinheiten repräsentiert.
Die Reste R³, R⁴, R⁵ und R⁶ bedeuten unabhängig voneinander -CH₃, -O-CH₃, -C₆H₅ oder -O-C₆H₅.
Die Indizes w und x repräsentieren stöchiometrische Koeffizienten, die unabhängig voneinander 0 bis 500, bevorzugt 10 bis 250, betragen.
Die Verteilung der Wiederholungseinheiten über die Kette hinweg kann nicht nur rein statistisch, sondern auch blockartig, alternierend oder gradientenartig.
R² kann einerseits einen aliphatischen, olefinischen, cycloaliphatischen, arylaliphatischen oder aromatischen (C₁ - C₅₀)-Kohlenwasserstoffrest symbolisieren (linear oder verzweigt) oder -OH, -NH₂, -N(CH₃)₂, -R⁷ oder für die Struktureinheit [-Z-R¹] stehen. Die Bedeutung der beiden Variablen Z und R¹ wurde bereits erklärt. R⁷ steht für weitere Si-haltige Gruppierungen. Bevorzugte R⁷-Reste sind -O-Si(CH₃)₃, -O-Si(Ph)₃, -O-Si(O-Si(CH₃)₃)₂CH₃) und -O-Si(O-Si(Ph)₃)₂Ph).
Wenn R² ein Element der Gruppe [-Z-R¹] darstellt, handelt es sich um difunktionelle, Monomere, die zur Vernetzung der entstehenden Polymerstrukturen herangezogen werden können.
Formel (I) beschreibt nicht nur vinylisch funktionalisierte, silikonhaltige Polymerspezies mit einer polymertypischen Verteilung, sondern auch definierte Verbindungen mit diskreten Molekulargewichten.

Besonders bevorzugte silikonhaltige Komponenten sind die folgenden acrylisch-oder methacrylisch modifizierten silikonhaltigen Komponenten: 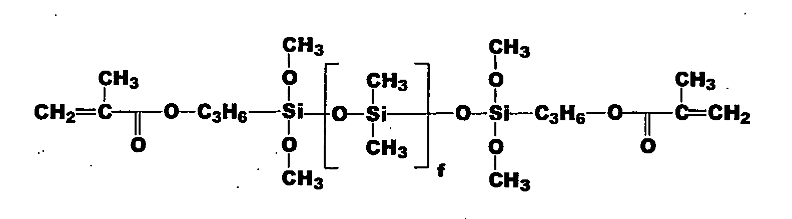 Methacryloxyproplydimethylsilyl endgeblockte Polydimethylsiloxane (f = 2 bis 500) 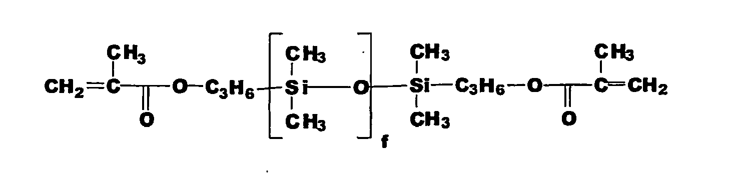 Methacryloxypropyl endgeblockte Polydimethylsiloxane (f = 2 bis 500 bis) 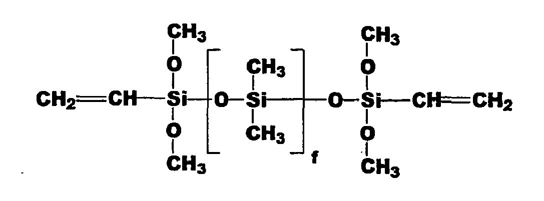 Vinyldimethoxysilyl endgeblockte Polydimethylsiloxane (f = 2-500)

Bezogen auf die Gesamtmasse der Copolymere kann der Gehalt an siliziumhaltigen Komponenten bis 99,9 Gew.-%, bevorzugt 0,5 bis 30 Gew.-%, insbesondere bevorzugt 1 bis 20 Gew.-%, betragen.

Als polymerisationsfähige, fluorhaltige Komponenten E) sind alle mindestens einfach olefinisch ungesättigten Verbindungen geeignet, die unter den jeweils gewählten Reaktionsbedingungen zur radikalischen Copolymerisation befähigt sind. Dabei muss die Verteilung der einzelnen fluorhaltigen Monomere über die entstehenden Polymerketten hinweg nicht notwendigerweise statistisch erfolgen. Auch die Ausbildung von beispielsweise block- (auch multiblock-) oder gradientenartigen Strukturen ist im Sinne der Erfindung. Kombinationen von zwei oder mehreren unterschiedlichen, fluorhaltigen Komponenten E) ist auch möglich, wobei dem Experten klar ist, dass monofunktionelle Vertreter zur Bildung kammförmiger Strukturen führen, wohingegen di-, tri-, oder polyfunktionelle Komponenten E) zu zumindest teilvernetzten Strukturen führen.

Bevorzugte fluorhaltige Komponenten E) sind solche gemäß Formel (II).

R¹-Y-CᵣH₂ᵣCₛF₂ₛCF₃ (II)

Dabei stellt R¹ eine polymerisationsfähige Funktion aus der Gruppe der vinylisch ungesättigten Verbindungen dar, die zum Aufbau polymerer Strukturen auf radikalischem Wege geeignet ist. Bevorzugt stellt R¹ ein Vinyl-, Allyl-, Methallyl-, Methylvinyl-, Acryl- (CH₂=CH-CO-), Methacryl- (CH₂=C[CH₃]-CO-), Crotonyl-, Senecionyl-, Itaconyl-, Maleinyl-, Fumaryl- oder Styrylrest, besonders bevorzugt einen Acryl- und Methacrylrest, dar.

Zur Anbindung der fluorhaltigen Gruppierung an die reaktive Endgruppe R¹ ist eine geeignete chemische Brücke Y erforderlich. Bevorzugte Brücken Y sind -O-, -C(O)-, -C(O)-O-, -S-, -O-CH₂-CH(O-)-CH₂OH, -O-CH₂-CH(OH)-CH₂-O-, -O-SO₂-O-, -O-S(O)-O-, -PH-, -P(CH₃)-, -PO₃-, -NH-, -N(CH₃)-, -O-(C₁-C₅₀)Alkyl-O-, -O-Phenyl-O-, -O-Benzyl-O-, -O-(C₅-C₈)Cycloalkyl-O-, -O-(C₁-C₅₀)Alkenyl-O-, -O-(CH(CH₃)-CH₂-O)ₙ-, -O-(CH₂-CH₂-O)ₙ- und -O-([CH-CH₂-O]ₙ-[CH₂-CH₂-O]ₘ)ₒ-, wobei n, m und o unabhängig voneinander Zahlen von 0 bis 200 bedeuten und die Verteilung der EO- und PO-Einheiten statistisch oder blockförmig sein kann.
Bei r und s handelt es sich um stöchiometrische Koeffizienten, die unabhängig voneinander Zahlen von 0 bis 200 bedeuten.

Bevorzugte fluorhaltige Komponenten E) gemäß Formel (II) sind
Perfluorhexylethanol-methacrylat,
Perfluorhexoylpropanol-methacrylat,
Perfluoroctyethanol-methacrylat,
Perfluoroctylpropanol-methacrylat,
Perfluorhexylethanolylpolygycolether-methacrylat,
Perfluorhexoyl-propanolyl-poly-[ethylglykol-co-propylenglycolether]-acrylat,
Perfluoroctyethanolyl-poly-[ethylglykol-blockco-propylenglycolether]-methacrylat,
Perfluoroctylpropanolyl-polypropylen-glycolether-methacrylat.

Bezogen auf die Gesamtmasse des Copolymeren kann der Gehalt an fluorhaltigen Komponenten bis 99.9 Gew.-%, bevorzugt 0.5 bis 30 Gew.-%, insbesondere bevorzugt 1 bis 20 Gew.-%, betragen.

Bei den Makromonomeren F) handelt sich um mindestens einfach olefinisch funktionalisierte Polymere mit einer oder mehreren diskreten Wiederholungseinheiten und einem zahlenmittleren Molekulargewicht größer oder gleich 200 g/mol. Bei der Copolymerisation können auch Mischungen chemisch unterschiedlicher Makromonomere F) eingesetzt werden. Bei den Makromonomeren handelt es sich um polymere Strukturen, die aus einer oder mehreren Wiederholungseinheit(en) aufgebaut sind und eine für Polymere charakteristische Molekulargewichtsverteilung aufweisen.

Die Makromonomere F) sind acrylisch- oder methacrylisch monofunktionalisierte Alkylethoxylate gemäß Formel. (IV). 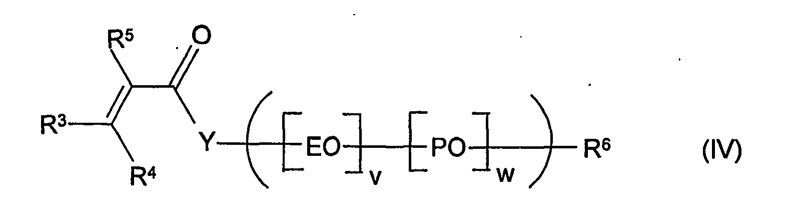 worin R₃ und R₄ gleich H oder -CH₃, besonders bevorzugt H; R₅ ist gleich H oder -CH₃; und R₆ ist gleich einem n-aliphatischen, iso-aliphatischen, olefinischen, cycloaliphatischen, arylaliphatischen oder aromatischen (C₁-C₃₀)-Kohlenwasserstoffrest, Y sind -O-, -C(O)-, -C(O)-O-, -S-, -O-CH₂-CH(O-)-CH₂OH, -O-CH₂-CH(OH)-CH₂O-, -O-SO₂-O-, -O-SO₂-O-, -O-SO-O-, -PH-, -P(CH₃)-, -PO₃-, -NH- und -N(CH₃)-, besonders bevorzugt -O-.
v und w sind wiederum die stöchiometrischen Koeffizienten betreffend die Ethylenoxideinheiten (EO) und Propylenoxideinheiten (PO). v und w betragen unabhängig voneinander 0 bis 500, bevorzugt 1 bis 30, wobei die Summe aus v und w im Mittel ≥ 1 sein muss. Die Verteilung der EO- und PO-Einheiten über die Makromonomerkette kann statistisch, blockartig, alternierend oder gradientenartig sein. Y steht für die obengenannten Brücken.

Weiterhin insbesondere bevorzugte Makromonomeren F) haben die folgende Struktur gemäß Formel (IV):

| Bezeichnung | R³ | R⁴ | R⁵ | R⁶ | v | w |
|---|---|---|---|---|---|---|
| ^{®}LA-030-methyacrylat | H | H | -CH₃ | -Lauryl | 3 | 0 |
| ^{®}LA-070-methacrylat | H | H | -CH₃ | -Lauryl | 7 | 0 |
| ^{®}LA-200-methacrylat | H | H | -CH₃ | -Lauryl | 20 | 0 |
| ^{®}LA-250-methacrylat | H | H | -CH₃ | -Lauryl | 25 | 0 |
| ^{®}T-080-methyacrylat | H | H | -CH₃ | -Talk | 8 | 0 |
| ^{®}T-080-acrylat | H | H | H | -Talk | 8 | 0 |
| ^{®}T-250-methyacrylat | H | H | -CH₃ | -Talk | 25 | 0 |
| ^{®}T-250-crotonat | -CH₃ | H | -CH₃ | -Talk | 25 | 0 |
| ^{®}OC-030-methacrylat | H | H | -CH₃ | -Octyl | 3 | 0 |
| ^{®}OC-105-methacrylat | H | H | -CH₃ | -Octyl | 10 | 5 |
| ^{®}Behenyl-010-methyaryl | H | H | H | -Behenyl | 10 | 0 |
| ^{®}Behenyl-020-methyaryl | H | H | H | -Behenyl | 20 | 0 |
| ^{®}Behenyl-010-senecionyl | -CH₃ | -CH₃ | H | -Behenyl | 10 | 0 |
| ^{®}PEG-440-diacrylat | H | H | H | -Acryl | 10 | 0 |
| ^{®}B-11-50-methacrylat | H | H | -CH₃ | -Butyl | 17 | 13 |
| ^{®}MPEG-750-methacrylat | H | H | -CH₃ | -Methyl | 18 | 0 |
| ^{®}P-010-acrylat | H | H | H | -Phenyl | 10 | 0 |
| ^{®}O-050-acrylat | H | H | H | -Oleyl | 5 | 0 |

Weiterhin als Makromonomere F) insbesondere geeignet sind Ester der (Meth)acrylsäure mit
(C₁₀-C₁₈)-Fettalkoholpolyglykolethern mit 8 EO-Einheiten (Genapol^{®} C-080)
C₁₁-Oxoalkoholpolyglykolethern mit 8 EO-Einheiten (Genapol^{®} UD-080)
(C₁₂-C₁₄)-Fettalkoholpolyglykolethern mit 7 EO-Einheiten (Genapol^{®} LA-070)
(C₁₂-C₁₄)-Fettalkoholpolyglykolethern mit 11 EO-Einheiten (Genapol^{®} LA-110)
(c₁₆-C₁₈)-Fettalkoholpolyglykolethern mit 8 EO-Einheiten (Genapol^{®} T-080)
(C₁₆-C₁₈)-Fettalkoholpolyglykolethern mit 15 EO-Einheiten (Genapol^{®} T-150)
(C₁₆-C₁₈)-Fettalkoholpolyglykolethern mit 11 EO-Einheiten (Genapol^{®} T-110)
(C₁₆-C₁₈)-Fettalkoholpolyglycolethern mit 20 EO-Einheiten (Genapol^{®} T-200)
(C₁₆-C₁₈)-Fettalkoholpolyglycolethern mit 25 EO-Einheiten (Genapol^{®} T-250)
(C₁₈-C₂₂)-Fettalkoholpolyglykolethern mit 25 EO-Einheiten und/oder
iso-(C₁₆-C₁₈)-Fettalkoholpolyglycolethem mit 25 EO-Einheiten
Bei den Genapol^{®}-Typen handelt es sich um Produkte der Firma Clariant, GmbH.

Bevorzugt beträgt das Molekulargewicht der Makromonomeren F) 200 g/mol bis 10⁶ g/mol, besonders bevorzugt 200 bis 10⁴ g/mol und insbesondere bevorzugt 200 bis 5000 g/mol.

Bezogen auf die Gesamtmasse der Copolymere können geeignete Makromonomere bis zu 99,9 Gew.-% eingesetzt werden. Bevorzugt finden die Bereiche 0,5 bis 30 Gew.-% und 70 bis 99,5 Gew.-% Anwendung. Besonders bevorzugt sind Anteile von 1 bis 20 Gew.-% und 75 bis 95 Gew.%.

Als Copolymere werden solche, die durch Copolymerisation mindestens der Komponenten A), C) und F) erhältlich sind, eingesetzt.

In einer bevorzugten Ausführungsform wird die Copolymerisation in Gegenwart mindestens eines polymeren Additivs G) durchgeführt, wobei das Additiv G) vor der eigentlichen Copolymerisation dem Polymerisationsmedium ganz- oder teilweise gelöst zugegeben wird. Die Verwendung von mehreren Additiven G) ist ebenfalls erfindungsgemäß. Vernetzte Additive G) können ebenfalls verwendet werden.
Die Additive G) bzw. deren Mischungen müssen lediglich ganz oder teilweise im gewählten Polymerisationsmedium löslich sein. Während des eigentlichen Polymerisationsschrittes hat das Additiv G) mehrere Funktionen. Einerseits verhindert es im eigentlichen Polymerisationsschritt die Bildung übervernetzter Polymeranteile im sich bildenden Copolymerisat und andererseits wird das Additiv G) gemäß dem allgemein bekannten Mechanismus der Pfropfcopolymerisation statistisch von aktiven Radikalen angegriffen. Dies führt dazu, dass je nach Additiv G) mehr oder weniger große Anteile davon in die Copolymere eingebaut werden.
Zudem besitzen geeignete Additive G) die Eigenschaft, die Lösungsparameter der sich bildenden Copolymere während der radikalischen Polymerisationsreaktion derart zu verändern, dass die mittleren Molekulargewichte zu höheren Werten verschoben werden. Verglichen mit analogen Copolymeren, die ohne den Zusatz der Additive G) hergestellt wurden, zeigen solche, die unter Zusatz von Additiven G) hergestellt wurden, vorteilhafterweise eine signifikant höhere Viskosität in wässriger Lösung.
Bevorzugt als Additive G) sind in Wasser und/oder Alkoholen, bevorzugt in t-Butanol, lösliche Homo- und Copolymere. Unter Copolymeren sind dabei auch solche mit mehr als zwei verschiedenen Monomertypen zu verstehen.
Besonders bevorzugt als Additive G) sind Homo- und Copolymere aus N-Vinylformamid, N-Vinylacetamid, N-Vinylpyrrolidon, Ethylenoxid, Propylenoxid, Acryloyldimethyltaurinsäure, N-Vinylcaprolactam, N-Vinylmethylacetamid, Acrylamid, Acrylsäure, Methacrylsäure, N-Vinylmorpholid, Hydroxyethylmethacrylat, Diallyldimethylammoniumchlorid (DADMAC) und/oder [2-(Methacryloyloxy)ethyl]trimethylammoniumchlorid (MAPTAC);
Polyalkylenglykole und/oder Alkylpolyglykole.
Insbesondere bevorzugt als Additive G) sind Polyvinylpyrrolidone (z.B. Luviskol K15^{®}, K20^{®} und K30^{®} von BASF), Poly(N-Vinylformamide), Poly(N-Vinylcaprolactame) und Copolymere aus N-Vinylpyrrolidon, N-Vinylformamid und/oder Acrylsäure, die auch teilweise oder vollständig verseift sein können.

Das Molekulargewicht der Additive G) beträgt bevorzugt 10² bis 10⁷ g/mol, besonders bevorzugt 0,5*10⁴ bis 10⁶ g/mol.
Die Einsatzmenge des polymeren Additivs G) beträgt, bezogen auf die Gesamtmasse der bei der Copolymerisation zu polymerisierenden Monomere, bevorzugt 0,1 bis 90 Gew.%, besonders bevorzugt 1 bis 20 Gew.% und insbesondere bevorzugt 1,5 bis 10 Gew.%.

In einer weiteren bevorzugten Ausführungsform sind die erfindungsgemäßen Copolymere vernetzt, d.h. sie enthalten Comonomere mit mindestens zwei polymerisationsfähigen Vinylgruppen.
Bevorzugte Vernetzer sind Methylenbisacrylamid; Methylenbismethacrylamid; Ester ungesättigter Mono- und Polycarbonsäuren mit Polyolen, bevorzugt Diacrylate und Triacrylate bzw. -methacrylate, besonders bevorzugt Butandiol- und Ethylenglykoldiacrylat bzw. -methacrylat, Trimethylolpropantriacrylat (TMPTA) und Trimethylolpropantrimethacrylat (TMPTMA); Allylverbindungen, bevorzugt Allyl(meth)acrylat, Triallylcyanurat, Maleinsäurediallylester, Polyallylester, Tetraallyloxyethan, Triallylamin, Tetraallylethylendiamin; Allylester der Phosphorsäure; und/oder Vinylphosphonsäurederivate.
Insbesondere bevorzugt als Vernetzer ist Trimethylolpropantriacrylat (TMPTA).
Der Gewichtsanteil an vernetzenden Comonomeren, bezogen auf die Gesamtmasse der Copolymere, beträgt bevorzugt bis 20 Gew.-%, besonders bevorzugt 0,05 bis 10 Gew.-% und insbesondere bevorzugt 0,1 bis 7 Gew.-%.

Als Polymerisationsmedium können alle organischen oder anorganischen Lösungsmittel dienen, die sich bezüglich radikalischer Polymerisationsreaktionen weitestgehend inert verhalten und vorteilhafterweise die Bildung mittlerer oder hoher Molekulargewichte zulassen. Bevorzugt Verwendung finden Wasser; niedere Alkohole; bevorzugt Methanol, Ethanol, Propanole, iso-, sec.- und t-Butanol, insbesondere bevorzugt t-Butanol; Kohlenwasserstoffe mit 1 bis 30 Kohlenstoffatomen und Mischungen der vorgenannten Verbindungen.

Die Polymerisationsreaktion erfolgt bevorzugt im Temperaturbereich zwischen 0 und 150°C, besonders bevorzugt zwischen 10 und 100°C, sowohl bei Normaldruck als auch unter erhöhtem oder erniedrigtem Druck. Gegebenenfalls kann die Polymerisation auch unter einer Schutzgasatmosphäre, vorzugsweise unter Stickstoff, ausgeführt werden.

Zur Auslösung der Polymerisation können energiereiche elektromagnetische Strahlen, mechanische Energie oder die üblichen chemischen Polymerisationsinitiatoren, wie organische Peroxide, z.B. Benzoylperoxid, tert.-Butylhydroperoxid, Methylethylketonperoxid, Cumolhydroperoxid, Dilauroylperoxid oder Azoinitiatoren, wie z.B. Azodiisobutyronitril (AIBN), verwendet werden.
Ebenfalls geeignet sind anorganische Peroxyverbindungen, wie z.B. (NH₄)₂S₂O_{8,} K₂S₂O₈ oder H₂O₂, gegebenenfalls in Kombination mit Reduktionsmitteln (z.B. Natriumhydrogensulfit, Ascorbinsäure, Eisen(II)-sulfat etc.) oder Redoxsystemen, welche als reduzierende Komponente eine aliphatische oder aromatische Sulfonsäure (z.B. Benzolsulfonsäure, Toluolsulfonsäure etc.) enthalten.

Als Polymerisationsmedium können alle Lösungsmittel dienen, die sich bezüglich radikalischer Polymerisationsreaktionen weitestgehend inert verhalten und die Bildung hoher Molekulargewichte zulassen. Bevorzugt Verwendung finden Wasser und niedere, tertiäre Alkohole oder Kohlenwasserstoffe mit 3 bis 30 C-Atomen. In einer besonders bevorzugten Ausführungsweise wird t-Butanol als Reaktionsmedium verwendet. Mischungen aus zwei- oder mehreren Vertretern der beschriebenen potentiellen Lösungsmitteln sind selbstverständlich ebenfalls erfindungsgemäß. Dies schließt auch Emulsionen von nicht miteinander mischbaren Solventien ein (z.B. Wasser/Kohlenwasserstoffe). Grundsätzlich sind alle Arten der Reaktionsführung geeignet, die zu den erfindungsgemäßen Polymerstrukturen führen (Lösungspolymerisation, Emulsionsverfahren, Fällungsverfahren, Hochdruckverfahren, Suspensionsverfahren, Substanzpolymerisation, Gelpolymerisation usw.).
Bevorzugt eignet sich die Fällungspolymerisation, besonders bevorzugt die Fällungspolymerisation in tert.-Butanol.

Die nachfolgende Auflistung zeigt 67 Copolymere. Die verschiedenen Copolymere Nr. 1 bis Nr. 67 sind gemäß den folgenden Herstellverfahren 1, 2, 3 und 4 erhältlich.

### Verfahren 1:

Diese Polymere sind nach dem Fällungsverfahren in tert. Butanol herstellbar.
Dabei wurden die Monomere in t-Butanol vorgelegt, die Reaktionsmischung inertisiert und anschließend die Reaktion nach Anheizen auf 60°C durch Zugabe des entsprechenden t-Butanol löslichen Initiators (bevorzugt Dilauroylperoxid) gestartet. Die Polymere werden nach beendeter Reaktion (2 Stunden) durch Absaugen des Lösungsmittels und durch anschließende Vakuumtrocknung isoliert.

### Verfahren 2:

Diese Polymere sind nach dem Gelpolymerisationsverfahren in Wasser herstellbar. Dabei werden die Monomere in Wasser gelöst, die Reaktionsmischung inertisiert und anschließend die Reaktion nach Anheizen auf 65°C durch Zugabe von geeigneten Initiatoren- oder Initiatorsystemen (bevorzugt Na₂S₂O₈) gestartet. Die Polymergele werden anschließend zerkleinert und nach Trocknung die Polymere isoliert.

### Verfahren 3:

Diese Polymere sind nach dem Emulsionsverfahren in Wasser herstellbar. Dabei werden die Monomere in einer Mischung aus Wasser/organ. Lösungsmittel (bevorzugt Cyclohexan) unter Verwendung eines Emulgators emulgiert, die Reaktionsmischung mittels N₂ inertisiert und anschließend die Reaktion nach Anheizen auf 80°C durch Zugabe von geeigneten Initiatoren- oder Initiatorsystemen (bevorzugt Na₂S₂O₈) gestartet. Die Polymeremulsionen werden anschließend eingedampft (Cyclohexan fungiert als Schlepper für Wasser) und dadurch die Polymere isoliert.

### Verfahren 4:

Diese Polymere sind nach dem Lösungsverfahren in organischen Lösungsmitteln (bevorzugt Toluol, z.B. auch tert. Alkohole) herstellbar. Dabei werden die Monomere im Lösungsmittel vorgelegt, die Reaktionsmischung inertisiert und anschließend die Reaktion nach Anheizen auf 70°C durch Zugabe von geeigneten Initiatoren- oder Initiatorsystemen (bevorzugt Dilauroylperoxid) gestartet. Die Polymere werden durch Abdampfen des Lösungsmittels und durch anschließende Vakuumtrocknung isoliert.

Polymere mit hydrophoben Seitenketten, unvernetzt
(außerhalb Anspruch 1)

| Nr. | Zusammensetzung | | Herstellverfahren |
|---|---|---|---|
| 1 | 95 g AMPS | 5 g Genapol T-080 | 1 |
| 2 | 90 g AMPS | 10 g Genapol T-080 | 1 |
| 3 | 85 g AMPS | 15 g Genapol T-080 | 1 |
| 4 | 80 g AMPS | 20 g Genapol T-080 | 1 |
| 5 | 70 g AMPS | 30 g Genapol T-080 | 1 |
| 6 | 50 g AMPS | 50 g Genapol T-080 | 3 |
| 7 | 40 g AMPS | 60 g Genapol T-080 | 3 |
| 8 | 30 g AMPS | 70 g Genapol T-080 | 3 |
| 9 | 20 g AMPS | 80 g Genapol T-080 | 3 |
| 10 | 60 g AMPS | 60 g BB10 | 4 |
| 11 | 80 g AMPS | 20 g BB10 | 4 |
| 12 | 90 g AMPS | 10 g BB10 | 3 |
| 13 | 80 g AMPS | 20 g BB10 | 1 |
| 14 | 80 g AMPS | 20 g Genapol LA040 | 1 |

Polymere mit hydrophoben Seitenketten, vernetzt
(außerhalb Anspruch 1)

| Nr. | Zusammensetzung | | | Herstellverfahren |
|---|---|---|---|---|
| 15 | 80 g AMPS | 20 g Genapol LA040 | 0.6g AMA | 1 |
| 16 | 80 g AMPS | 20 g Genapol LA040 | 0,8 AMA | 1 |
| 17 | 80 g AMPS | 20 g Genapol LA040 | 1,0 g AMA | 1 |
| 18 | 628,73 g AMPS | 120,45 g Genapol T-250 | 6,5 g TMPTA | 2 |
| 19 | 60 g AMPS | 40 g BB10 | 1,9 g TMPTA | 4 |
| 20 | 80 g AMPS | 20 g BB10 | 1,4 g TMPTA | 4 |
| 21 | 90 g AMPS | 10 g BB10 | 1,9 g TMPTA | 4 |
| 22 | 80 g AMPS | 20 g BB10 | 1,9 g TMPTA | 4 |
| 23 | 60 g AMPS | 40 g BB10 | 1,4 g TMPTA | 4 |

Polymere mit hydrophoben Seitenketten, vernetzt, gepfropft (außerhalb Anspruch 1)

| Nr. | Zusammensetzung | | | | Herstellverfahren |
|---|---|---|---|---|---|
| 24 | 95 g AMPS | 5 g BB10, | 1,9 g TMPTA, | 1 g Poly-NVP | 1 |
| 25 | 90 g AMPS | 10 g BB10, | 1,9 g TMPTA, | 1 g Poly-NVP | 1 |
| 26 | 85 g AMPS | 15 g BB10, | 1,9 g TMPTA, | 1 g Poly-NVP | 1 |
| 27 | 90 g AMPS | 10 g BB10, | 1,9 g TMPTA, | 1 g Poly-NVP | 1 |

Polymere mit siliziumhaltigen Gruppen, unvernetzt
(außerhalb Anspruch 1)

| Nr. | Zusammensetzung | | Herstellverfahren |
|---|---|---|---|
| 28 | 80 g AMPS, | 20 g Silvet 867 | 1 |
| 29 | 80 g AMPS, | 50 g Silvet 867 | 4 |

Polymere mit siliziumhaltigen Gruppen, vernetzt
(außerhalb Anspruch 1)

| Nr. | Zusammensetzung | | Herstellverfahren |
|---|---|---|---|
| 30 | 80 g AMPS, 20 g Silvet 867, | 0,5 g MBA | 4 |
| 31 | 80 g AMPS, 20 g Silvet 867, | 1,0 g MBA | 1 |
| 32 | 60 g AMPS, 40 g Y-12867, | 0,95 g AMA | 1 |
| 33 | 80 g AMPS, 20 g Y-12867, | 0,95 g AMA | 1 |
| 34 | 90 g AMPS, 10 g Y-12867, | 0,95 g AMA | 1 |
| 35 | 60 g AMPS, 40 g Silvet 7280, | 0,95 g AMA | 1 |
| 36 | 80 g AMPS, 20 g Silvet 7280, | 0,95 g AMA | 1 |
| 37 | 90 g AMPS, 10 g Silvet 7280, | 0,95 g AMA | 1 |
| 38 | 60 g AMPS, 40 g Silvet 7608, | 0,95 g AMA | 1 |
| 39 | 80 g AMPS, 20 g Silvet 7608, | 0,95 g AMA | 1 |
| 40 | 90 g AMPS, 10 g Silvet 7608, | 0,95 g AMA | 1 |

Polymere mit hydrophoben Seitenketten und kationischen Gruppen, unvernetzt
(außerhalb Anspruch 1)

| Nr. | Zusammensetzung | | | Herstellverfahren |
|---|---|---|---|---|
| 41 | 87,5 g AMPS, | 7,5 g Genapol T-110, | 5 g DADMAC | 2 |
| 42 | 40 g AMPS, | 10 g Genapol T110, | 45 g Methacrylamid | 2 |
| 43 | 55 g AMPS, | 40 g Genapol LA040, | 5 g Quat | 1 |
| 44 | 75 g AMPS, | 10 g BB10, | 6,7 g Quat | 1 |

Polymere mit hydrophoben Seitenketten und kationischen Gruppen, vernetzt
(außerhalb Anspruch 1)

| Nr. | Zusammensetzung | | | | Herstellverfahren |
|---|---|---|---|---|---|
| 45 | 60 g AMPS, | 20 g Genapol T-80, | 10 g Quat, | 10 g HEMA | 1 |
| 46 | 75 g AMPS, | 20 g GenapolT-250, | 5 g Quat, | 1,4 g TMPTA | 1 |
| 47 | 75 g AMPS, | 20 g GenapolT-250, | 10 g Quat, | 1,4 g TMPTA | 1 |
| 48 | 75 g AMPS, | 20 g GenapolT-250, | 20 g Quat, | 1,4 g TMPTA | 1 |

Polymere mit fluorhaltigen Gruppen
(außerhalb Anspruch 1)

| Nr. | Zusammensetzung | Herstellverfahren |
|---|---|---|
| 49 | 94 g AMPS, 2,02 g Fluowet AC 600 | 1 |
| 50 | 80 g AMPS, | 3 |
| | 20 g Perfluoroctylpolyethylenglykolmethacrylat, 1 g Span 80 | |

Polymere mit fluorhaltigen Gruppen, gepfropft
(außerhalb Anspruch 1)

| Nr. | Zusammensetzung | Herstellverfahren |
|---|---|---|
| 51 | 80 g AMPS, 10 g Fluowet AC 600, 5 g Poly-NVP | 1 |
| 52 | 70 g AMPS, 8 g Perfluoroctylethyloxyglycerinmethacrylat, 5 g Poly-NVP | 4 |

### Multifunktionelle Polymere

| Nr. | Zusammensetzung | Herstellverfahren |
|---|---|---|
| 53* | 80 g AMPS, 10 g Genapol LA070, 10 g Silvet 7608, 1,8 g TMPTA | 1 |
| 54 | 70 g AMPS, 5 g N-Vinylpyrrolidon, 15 g Genapol T-250 Methacrylat, 10 g Quat, 10 g Poly-NVP | 4 |
| 55 | 80 g AMPS, 5 g N-Vinylformamid, 5 g Genapol O-150- methacrylat, 10 g DADMAC, 1,8 g TMPTA, 8 g Poly-N-Vinylformamid | 2 |
| 56 | 70 g AMPS, 5 g N-Vinylpyrrolidon, 15 g Genapol T-250-methacrylat, 10 g Quat, 10 g Poly-NVP | 1 |
| 57 | 60 g AMPS, 10 g Genapol-BE-020-methacrylat, 10 g Genapol T-250-acrylat, 20 g Quat, 1 g Span 80 | 1 |
| 58 | 60 g AMPS, 20 g MPEG-750-methacrylat, 10 g Methacryloxypyldimethicon, 10 g Perfluorooctylpolyethylenglycol-methacrylat, 10 g Poly[N-vinylcaprolacton-co-acrylsäure] (10/90) | 1 |
| 59 | 80 g AMPS, 5 g N-Vinylformamid, 5 g Genapol O-150-methacrylat, 10 g DADMAC, 1,8gTMPTA | 1 |
| 60 | 70 g AMPS, 10 g Genapol T-250-acrylat, 5 g N-Methyl-4-vinylpyridiniumchlorid, 2,5 g Silvet Y-12867, 2,5 g Perfluorhexylpolyethylenglykolmethacrylat, 10 g Polyethylenglykoldimethacrylat, 4 g Poly[N-Vinylcaprolactam] | 1 |
| 61* | 10 g AMPS, 20 g Acrylamid, 30 g N-2-Vinylpyrrolidon, 20 g Silvet 7608, 10 g Methacryloxypyl dimethicon, 10 g Fluowet AC 812 | 3 |
| 62 | 60 g AMPS, 10 g DADMAC, 10 g Quat, 10 g Genapol-LA-250-crotonat, 10 g Methacryloxypyldimethicon, 7 g Poly[acrylsäure-co-N-vinylformamid] | 1 |
| 63* | 50 g AMPS, 45 g Silvet 7608, 1,8 g TMPTA, 8 g Poly[N-Vinylformamid] | 1 |
| 64* | 20 g AMPS, 10 g Genapol T 110, 35 g MAA, 30 g * HEMA, 5 g DADMAC | 4 |
| 65* | 20 g AMPS, 80gBB10, 1,4 g TMPTA | 1 |
| 66* | 75 g AMPS, 20 g BB10, 6,7 g Quat, 1,4 g TMPTA | 1 |
| 67* | 35 g AMPS, 60 g Acrylamid, 2 g VIFA, 2,5 g Vinylphosphonsäure, 2 Mol-% Fluowet EA-600 | 4 |

| | | |
|---|---|---|
| (* auberhalß Anspruch 1) | | |

### Chemische Bezeichnung der Reaktanden:

| | |
|---|---|
| AMPS | Acryloyldimethyltaurat, wahlweise Na- oder NH4-Salz |
| Genapol^{®} T-080 | C₁₆-C₁₈-Fettalkoholpolyglykolether mit 8 EO-Einheiten |
| Genapol^{®} T-110 | C₁₂-C₁₄-Fettalkoholpolyglykolether mit 11 EO -Einheiten |
| Genapol^{®} T-250 | C₁₆-C₁₈-Fettalkoholpolyglycolether mit 25 EO-Einheiten |
| Genapol^{®} LA-040 | C₁₂-C₁₄-Fettalkoholpolyglykolether mit 4 EO-Einheiten |
| Genapol^{®} LA-070 | C₁₂-C₁₄-Fettalkoholpolyglykolether mit 7 EO-Einheiten |
| Genapol^{®} O-150 methacrylat | C₁₆-C₁₈-Fettalkoholpolyglykolether Methacrylat mit 15 EO-Einheiten, |
| Genapol^{®} LA-250 crotonat | C₁₂-C₁₄-Fettalkoholpolyglykolether crotonat mit 25 EO-Einheiten |
| Genapol^{®} T-250 methacrylat | C₁₆-C₁₈-Fettalkoholpolyglycolether methacrylat mit 25 EO-Einheiten |
| Genapol^{®} T-250 acrylat | C₁₆-C₁₈-Fettalkoholpolyglycolether methacrylat mit 25 EO-Einheiten |
| BB10^{®} | Polyoxyethylen(10)Behenylether |
| TMPTA | Trimethylolpropantriacrylat |
| Poly-NVP | Poly-N-Vinylpyrrolidon |
| Silvet^{®} 867 | Siloxan Polyalkylenoxid Copolymer |
| MBA | Methylen-bis-acrylamid |
| AMA | Allylmethacrylat |
| ^{®}Y-12867 | Siloxan Polyalkylenoxid Copolymer |
| Silvet^{®} 7608 | Polyalkylenoxid modifiziertes Heptamethyltrisiloxan |
| Silvet^{®} 7280 | Polyalkylenoxid-modifiziertes Heptamethyltrisiloxan |
| DADMAC | Diallyldimethyl-ammoniumchlorid |
| HEMA | 2-Hydroxyethylmethacrylat |
| Quat | 2-(Methacryloyloxy)ethyltrimethylammoniumchlorid |
| Fluowet^{®} AC 600 | Perfluoralkylethylacrylat |
| Span^{®} 80 | Sorbitanester |

In einer bevorzugten Ausführungsform sind die Copolymere wasserlöslich oder wasserquellbar.
Die beschriebene, optional durchführbare Pfropfung der Copolymere mit anderen Polymeren führt zu Produkten mit besonderer Polymermorphologie, die in wässrigen Systemen optisch klare Gele ergeben. Ein potenzieller Nachteil der Copolymere ohne Pfropfung besteht in einer mehr oder weniger starken Opaleszenz in wässriger Lösung. Diese beruht auf bisher nicht zu vermeidenden, übervernetzten Polymeranteilen, die während der Synthese entstehen und in Wasser nur unzureichend gequollen vorliegen. Dadurch bilden sich Licht streuende Teilchen aus, deren Größe deutlich oberhalb der Wellenlänge des sichtbaren Lichts liegt und deshalb Ursache der Opaleszenz sind. Durch das beschriebene, optional durchführbare Pfropfverfahren wird die Bildung übervernetzter Polymeranteile gegenüber herkömmlichen Techniken deutlich reduziert oder gänzlich vermieden.

Die beschriebene Inkorporation sowohl von kationischen Ladungen als auch von Silizium-, Fluor oder Phosphoratomen in die Copolymere führt zu Produkten, die in kosmetischen Formulierungen besondere sensorische und rheologische Eigenschaften besitzen. Eine Verbesserung der sensorischen und rheologischen Eigenschaften kann insbesondere bei der Verwendung in Rinse-off Produkten vorteilhaft sein.

Siliziummodifizierte Copolymere können die Funktionen von Silikonölen in teilweise oder in vollem Umfang übernehmen. Der Einsatz von Silikonen kann durch die Copolymere reduziert oder vermieden werden.

Die Haarbehandlungsmittel enthalten, bezogen auf die fertigen Mittel, bevorzugt 0,01 bis 10 Gew.-%, besonders bevorzugt 0,1 bis 5 Gew.-%, insbesondere bevorzugt 0,5 bis 3 Gew.-% der Copolymere.

Die Haarbehandlungsmittel können als weitere Hilfs- und Zusatzstoffe Ölkörper, Emulgatoren und Co-Emulgatoren, kationische Polymere, Filmbildner, sowie weitere in der Kosmetik gebräuchliche Zusätze, wie z.B. Überfettungsmittel, feuchtigkeitsspendende Mittel, Stabilisatoren, biogene Wirkstoffe, Glycerin, Konservierungsmittel, Perlglanzmittel, Farb- und Duftstoffe, Lösungsmittel, Hydrotrope, Trübungsmittel, weitere Verdickungsmittel und Dispergiermittel, ferner Eiweißderivate, wie Gelatine, Collagenhydrolysate, Polypeptide auf natürlicher und synthetischer Basis, Eigelb, Lecithin, Lanolin und Lanolinderivate, Fettalkohole, Silicone, Stoffe mit keratolytischer und keratoplastischer Wirkung, Enzyme und Trägersubstanzen, Antioxidation, Lichtschutzstoffe UV-Lichtschutzfilter, biogene Wirkstoffe (Lokalanästhetika, Antibiotika, Antiphlogistik, Antiallergica, Corticosteroide, Sebostatika) pharmazeutisch aktive Wirkstoffe und/oder Antischuppenmittel enthalten.

Unter Ölkörper ist jegliche Fettsubstanz zu verstehen, die bei Raumtemperatur (25°C) flüssig ist.
Die Fett-Phase kann daher ein oder mehrere Öle umfassen, die vorzugsweise aus folgenden Ölen ausgewählt werden:
Silikonöle, flüchtig oder nicht flüchtig, linear, verzweigt oder ringförmig, eventuell organisch modifiziert; Phenylsilikone; Silikonharze und -gummis; Mineralöle wie Paraffin- oder Vaselinöl; Öle tierischen Ursprungs wie Perhydrosqualen, Lanolin; Öle pflanzlichen Ursprungs wie flüssige Triglyceride, z.B. Sonnenblumen-, Mais-, Soja-, Reis-, Jojoba-, Babusscu-, Kürbis-, Traubenkern-, Sesam-, Walnuss-, Aprikosen-, Makadamia-, Avocado-, Süßmandel-, Wiesenschaumkraut-, Ricinusöl, Triglyceride der Capryl/Caprinsäuren, Olivenöl, Erdnussöl, Rapsöl und Kokosnussöl;
Synthetische Öle wie Purcellinöl, Isoparaffine, lineare und/oder verzweigte Fettalkohole und Fettsäureester, bevorzugt Guerbetalkohole mit 6 bis 18, vorzugsweise 8 bis 10, Kohlenstoffatomen; Ester von linearen (C₆-C₁₃)-Fettsäuren mit linearen (C₆-C₂₀)-Fettalkoholen; Ester von verzweigten (C₆-C₁₃)-Carbonsäuren mit linearen (C₆-C₂₀)-Fettalkoholen, Ester von linearen (C₆-C₁₈)-Fettsäuren mit verzweigten Alkoholen, insbesondere 2-Ethylhexanol; Ester von linearen und/oder verzweigten Fettsäuren mit mehrwertigen Alkoholen (wie z.B. Dimerdiol oder Trimerdiol) und/oder Guerbetalkoholen; Triglyceride auf Basis (C₆-C₁₀)-Fettsäuren;
Ester wie Dioctyladipat, Diisopropyl dimer dilineloat; Propylenglycole/-dicaprilat oder Wachse wie Bienenwachs, Paraffinwachs oder Mikrowachse, gegebenenfalls in Kombination mit hydrophilen Wachsen, wie z.B. Cetylstearylalkohol; Fluorierte und perfluorierte Öle; fluorierte Silikonöle; Gemische der vorgenannten Verbindungen.

Als nichtionogene Co-Emulgatoren kommen u.a. in Betracht Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; (C₁₂-C₁₈)-Fettsäuremono- und -diester von Anlagerungsprodukten von 0 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und ggfs. deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z.B. Polyglycerinpolyricinoleat und Polyglycerinpoly-12-hydroxystearat.Ebenfalls geeignet sind Gemische von Verbindungen aus mehreren dieser Substanzklassen. Als ionogene Co-Emulgatoren eignen sich z.B. anionische Emulgatoren, wie mono-, di- oder tri-Phosphorsäureester, aber auch kationische Emulgatoren wie mono-, di- und tri-Alkylquats und deren polymere Derivate.

Als kationische Polymere eignen sich die unter der INCI-Bezeichnung "Polyquaternium" bekannten Verbindungen, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, Polyquaternium 37&mineral oil&PPG trideceth (^{®}Salcare SC95), PVP-dimethylaminoethylmethacrylat-Copolymer, Guarhydroxypropyltriammoniumchloride, sowie Calciumalginat und Ammoniumalginat.
Des weiteren können eingesetzt werden kationische Cellulosederivate; kationische Stärke; Copolymere von Diallylammoniumsalzen und Acrylamiden; quaternierte Vinylpyrrolidon/ Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z.B. Amidomethicone; Copolymere der Adipinsäure und Dimethylaminohydroxy-propyldiethylentriamin; Polyaminopolyamid und kationische Chitinderivate, wie beispielsweise Chitosan. Geeignete Siliconverbindungen sind beispielsweise Dimethylpolysiloxan, Methylphenylpolysiloxane, cyclische Silicone und amino-, fettsäure-, alkohol-, polyether-, epoyx-, fluor- und/oder alkylmodifizierte Siliconverbindungen, sowie Polyalkylsiloxane, Polyalkylarylsiloxane, Polyethersiloxan-Copolymere, wie in US 5104 645 und den darin zitierten Schriften beschrieben, die bei Raumtemperatur sowohl flüssig als auch harzförmig vorliegen können.
Geeignete Filmbildner sind je nach Anwendungszweck Salze der Phenylbenzimidazolsulfonsäure, wasserlösliche Polyurethane, beispielsweise C₁₀-Polycarbamylpolyglycerylester, Polyvinylalkohol, Polyvinylpyrrolidon, -copolymere, beispielsweise Vinylpyrrolidon/Vinylacetatcopolymer, wasserlösliche Acrylsäurepolymere/ Copolymere bzw.deren Ester oder Salze, beispielsweise Partialestercopolymere der Acryl/ Methacrylsäure und Polyethylenglykolether von Fettalkoholen, wie Acrylat/Steareth-20-Methacrylat Copolymer, wasserlösliche Cellulose, beispielsweise Hydroxymethylcellulose, Hydroxyethylcellulose, Hydroxypropylcellulose, wasserlösliche Quaterniums, Polyquaterniums, Carbocyvinyl-Polymere, wie Carbomere und deren Salze, Polysaccharide, beispielsweise Polydextrose und Glucan.

Als Überfettungsmittel können Substanzen wie beispielsweise Lanolin und Lecithin, nicht ethoxylierte und polyethoxylierte oder acylierte Lanolin- und Lecithinderivate, Polyolfettsäureester, Mono-, Di- und Triglyceride und/oder Fettsäurealkanolamide verwendet werden.

Als feuchtigkeitsspendende Substanz stehen beispielsweise Isopropylpalmitat, Glycerin und/ oder Sorbitol zu Verfügung.

Als Stabilisatoren können Metallsalze von Fettsäuren, wie z.B. Magnesium-, Aluminium- und/oder Zinkstearat eingesetzt werden.
Unter biogenen Wirkstoffen sind beispielsweise Pflanzenextrakte und Vitaminkomplexe zu verstehen.

Zusätzlich können die erfindungsgemäßen Mittel organische Lösungsmittel enthalten. Prinzipiell kommen als organische Lösungsmittel alle ein- oder mehrwertigen Alkohole in Betracht. Bevorzugt werden Alkohole mit 1 bis 4 Kohlenstoffatomen wie Ethanol, Propanol, Isopropanol, n-Butanol, i-Butanol, t-Butanol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45 Gew.-% und von Polyethylenglykol mit einer relativen Molekülmasse zwischen 400 und 600 in Mengen von 5 bis 25 Gew.-% bevorzugt. Weitere geeignete Lösungsmittel sind beispielsweise Triacetin (Glycerintriacetat) und 1-Methoxy-2-propanol. Hydrotrop wirken kurzkettige Aniontenside, insbesondere Arylsulfonate, beispielsweise Cumol- oder Toluolsulfonat.

Die erfindungsgemäßen Mittel können mit konventionellen Ceramiden, Pseudoceramiden, Fettsäure-N-alkylpolyhydroxyalkylamiden, Cholesterin, Cholesterinfettsäureestern, Fettsäuren, Triglyceriden, Cerebrosiden, Phospholipiden und ähnlichen Stoffen als Pflegezusatz abgemischt werden.
Als UV-Filter eignen sich z.B. 4-Aminobenzoesäure;
3-(4'-Trimethylammonium)benzyliden-boran-2-on-methylsulfat;
3,3,5-Trimethyl-cyclohexylsalicylat; 2-Hydroxy-4-methoxybenzophenon;
2-Phenylbenzimidazol-5-sulfonsäure und ihre Kalium-, Natrium- und
Triethanolaminsalze; 3,3'-(1,4-Phenylendimethin)-bis-(7,7-dimethyl-2-oxobicyclo[2.2.1]-heptan-1-methansulfonsäure und ihre Salze; 1-(4-tert.-Butylphenyl)-3-(4-methoxyphenyl)propan-1,3-dion, 3-(4'-Sulfo)-benzyliden-bornan-2-on und seine Salze; 2-Cyan-3,3-diphenyl-acrylsäure-(2-ethylhexylester); Polymer von N-[2(und 4)-(2-oxoborn-3-ylidenmethyl)benzyl]-acrylamid; 4-Methoxyzimtsäure-2-ethyl-hexylester; ethoxyliertes Ethyl-4-amino-benzoat; 4-Methoxyzimtsäure-isoamylester; 2,4,6-Tris-[p-(2-ethylhexyloxycarbonyl)anilino]-1,3,5-triazin;
2-(2H-benzotriazol-2-yl)-4-methyl-6-(2-methyl-3-(1,3,3,3-tetramethyl-1-(trimethylsilyloxy)-disiloxanyl)propyl)phenol;
4,4'-[(6-[4-((1,1-dimethylethyl)-amino-carbonyl)phenylamino]-1,3,5-triazin-2,4-yl)diimino]bis-(benzoesäure-2-ethylhexylester);
3-(4'-Methylbenzyliden)-D,L-Campher; 3-Benzyliden-Campher; Salicylsäure-2-ethylhexylester; 4-Dimethylaminobenzoesäure-2-ethylhexylester; Hydroxy-4-methoxy-benzophenon-5-sulfonsäure (Sulisobenzonum) und das Natriumsalz; und/oder 4-Isopropylbenzylsalicylat.
Als Antioxidantien eignen sich beispielsweise Superoxid-Dismutase, Tocopherol (Vitamin E) und Ascorbinsäure (Vitamin C).

Als Konservierungsmittel in Betracht kommen beispielsweise Phenoxyethanol, Parabene, Pentandiol oder Sorbinsäure.

Als Farbstoffe können die für kosmetische Zwecke geeigneten und zugelassenen Substanzen verwendet werden.
Als Antischuppenmittel bzw. antifungizide Wirkstoffe eignen sich bevorzugt Ketoconazol, Climbazol^{®}, Octopirox^{®}, Oxiconazol, Terbinafin, Bifonazole, Butoconazole, Cloconazole, Clotrimazole, Econazole, Enilconazole, Fenticonazole, Isoconazole, Miconazole, Sulconazole, Tioconazole, Fluconazole, Itraconazole, Terconazole, Naftifine und Terbinafine, Zn-Pyrethion und Oczopyrox.

Als biogene Wirkstoffen können beispielsweise Bisabolol^{®}, Allantoin^{®}, Phytantriol^{®}, Panthenol^{®}, AHA-Säuren, Pflanzenextrakte und Vitaminkomplexe eingesetzt werden.

Als Säure bzw. Laugen zur pH-Wert Einstellung werden bevorzugt Zitronensäure und/oder Natronlauge verwendet.

Die Mittel sind üblicherweise auf einen pH Wert im Bereich 2 bis 12, bevorzugt pH 3 bis 8, eingestellt.

Der Gesamtanteil an Hilfs- und Zusatzstoffen in den Haarbehandlungsmitteln beträgt bevorzugt 1 bis 30 Gew.-%, besonders bevorzugt 2 bis 20 Gew.-%.

Bevorzugte Ausführungsformen sind Spülungen, Kuren, Sprühkuren, Lotionen, Cremes, Gele, Schäume und Sprays

Die nachfolgenden Beispiele und Anwendungen sollen die Erfindung näher erläutern, ohne sie jedoch darauf zu beschränken (bei allen Prozentangaben handelt es sich um Gewichtsprozent). Bei den in den Beispielen verwendeten Copolymeren handelt es sich um Vertreter der in der Beschreibung bereits aufgeführten Copolymere Nr.1 bis Nr.67. Die Herstellung erfolgte nach den dort angegebenen Verfahren 1, 2, 3 oder 4 unter Verwendung der bevorzugten Initiatoren und Lösemittel.

### Referenz-Beispiel 1: Haarspülung

| | | |
|---|---|---|
| I) | Genaminox CSL | 6,0 % |
| | Cetiol HE | 2,0 % |
| II) | Copolymer Nr. 48 | 1,2 % |
| III) | Wasser | ad 100 % |

Zur Herstellung der Haarspülungen 1 wird jeweils eine Mischung 1 aus den Komponenten unter I) hergestellt. Dazu werden die Komponenten unter I) bei ca. RT unter Rühren bis zum Klarwerden gelöst. Anschließend wird die Mischung 1 auf Raumtemperatur abgekühlt. Zur Herstellung einer Mischung 2 wird jeweils die Komponente II) in der Komponente III) dispergiert und die Mischung bis zum Klarwerden gerührt. Anschließend werden die Mischungen 1 und 2 unter Rühren miteinander vermischt. Danach wird der pH-Wert mittels Zitronensäure auf ca. pH=4 eingestellt.

### Referenz-Beispiel 2: Haarspülung

| | | |
|---|---|---|
| I) | Cetylalkohol | 3,0% |
| | Hostaphat KL 340 D | 1,5 % |
| | Paraffinöl nv | 0,5% |
| II) | Copolymer Nr.35 | 1,0 % |
| IV) | Wasser | ad 100 % |

Zur Herstellung der Haarspülung 2 werden die Komponenten unter I bei ca. 75°C aufgeschmolzen (Mischung 1). II) wird unter Rühren in III) aufgequollen und anschließend auf ca. 75°C erhitzt (Mischung 2). Danach wird die Mischung 2 zur Mischung 1 unter Rühren zugegeben. Unter Rühren auf Raumtemperatur abkühlen.
Abschließend pH- Wert auf ca. 4 einstellen.

### Referenz-Beispiele 3: Haarsprühkur

| | | |
|---|---|---|
| I) | Copolymer Nr. 49 | 2 % |
| | Genaminox CS | 4% |
| | Cetiol HE | 2% |
| | Panthenol | 0,2 % |
| II) | Wasser | ad 100 % |

Zur Herstellung der Haarsprühkur wird jeweils eine Mischung aus den Komponenten I) und II) hergestellt. Dazu werden die Komponenten unter I) unter Rühren in der Komponente II) bis zum Klarwerden gelöst. Anschließend wird die Mischung auf Raumtemperatur abgekühlt. Danach wird der pH-Wert mittels Zitronensäure auf ca. pH=4 eingestellt.

### Beispiel 4: Haarkur

| | |
|---|---|
| Genamin KSL | 7% |
| Hostaphat KL 340 D^{®} | 1,5% |
| Genapol PDC^{®} | 4% |
| Copolymer Nr. 60 | 1,7% |
| Paraffinöl nv | 1 % |
| Jojobaöl | 1 % |
| Propylenglykol | 0,8 % |
| Isopropylpalmitat | 1 % |
| Dow Corning 190^{®} | 0,8% |
| Extrapon | 0,3% |
| Vitamin E | 0,3 % |
| Panthenol (Vitamin B 5) | 0,5 % |

Die Herstellung erfolgt in den aufeinanderfolgenden Schritten I bis VI:

| | |
|---|---|
| I | Polymer im Wasser bei RT unter Rühren aufquellen |
| II | Ölphase, enthaltend Öl/e, Quats, Lösungsvermittler und gegebenenfalls Vitamine bei ca. 75°C aufschmelzen |
| III | Wasserphase (I) auf ca. 75°C erhitzen |
| IV | Wasserphase (I) zur Ölphase (II) geben und kaltrühren |
| V | Bei ca. 30°C Perlglanzkonzentrat, evt. Farbstoff, Parfüm und Pflanzenextrakte zugeben |
| VI | pH-Wert einstellen |

### Referenz-Beispiel 5: Haarspülung

| | | |
|---|---|---|
| I) | Genamin CTAC | 5,0 % |
| | Genamin KDMP | 0,5 % |
| | Genaminox LA | 5,0 % |
| | Velsan D8P-3 | 1,0 % |
| II) | Wasser | 27,3 % |
| III) | Copolymer Nr.41 | 1,2 % |
| IV) | Wasser | ad 100 % |

Zur Herstellung der Haarspülungen aus den Beispielen 1 bis 3 wird jeweils eine Mischung 1 aus den Komponenten I) und II) hergestellt. Dazu wird die Komponente I) bei ca. 60°C unter Rühren in der Komponente II) bis zum Klarwerden gelöst. Anschließend wird die Mischung 1 auf Raumtemperatur abgekühlt. Zur Herstellung einer Mischung 2 wird jeweils die Komponente III) in der Komponente IV) dispergiert und die Mischung bis zum Klarwerden gerührt. Anschließend werden die Mischungen 1 und 2 unter Rühren miteinander vermischt. Danach wird der pH-Wert mittels Zitronensäure auf ca. pH=4 eingestellt.

### INCI Bezeichnung der eingesetzten Handelsprodukte:

| | | |
|---|---|---|
| Genamin KSL^{®} | (Clariant) | PEG-5 Stearyl Ammonium Lactat |
| Genapol PDC^{®} | (Clariant) | Glycol Distearat (and) Laureth- 4 (and) Cocamidopropyl Betain (and) Mica (and) Titanium Dioxide |
| Hostaphat KL 340 D^{®} | (Clariant) | Trilaureth-4 Phosphat |
| Dow Corning 190^{®} | (Dow Corning) | Dimethicon Copolyol |
| Extrapon^{®} | (Dragoco) | Pflanzliche Extrakte |
| Genaminox LA | (Clariant GmbH) | Lauryldimethylaminoxid |
| Genaminox CSL | (Clariant GmbH) | Cocaminoxid |
| Genapol UD-80 | (Clariant GmbH) | Undeceth-8 |
| Velsan D8P-3 | (Clariant GmbH) | Isopropyl PPG-2-Isodeceth-7 Carboxylat |
| Cetiol HE | (Henkel) | PEG-7 Glyceryl Cocoat |
| Genagen CA-050 | (Clariant GmbH) | PEG-5 Cocamid |
| Genamin KDM-P^{®} | (Clariant) | Behenyltrimethylammonium Chlorid |
| Genamin CTAC^{®} | (Clariant) | Cetyltrimethylammonium Chlorid |

## Patentansprüche

1. Kosmetische und dermatologische Haarbehandlungsmittel, **dadurch gekennzeichnet, dass** sie mindestens ein Copolymer, erhältlich durch radikalische Copolymerisation von
A) Acryloyldimethyltaurinsäure und/oder Acryloyldimethyltauraten,
B) gegebenenfalls einem oder mehreren weiteren olefinisch ungesättigten, nicht kationischen, gegebenenfalls vernetzenden, Comonomeren, die wenigstens ein Sauerstoff-, Stickstoff-, Schwefel- oder Phosphoratom aufweisen und ein Molekulargewicht kleiner 500 g/mol besitzen,
C) einem oder mehreren olefinisch ungesättigten, kationischen Comonomeren, die wenigstens ein Sauerstoff-, Stickstoff-, Schwefel- oder Phosphoratom aufweisen und ein Molekulargewicht kleiner 500 g/mol besitzen,
D) gegebenenfalls einer oder mehreren mindestens monofunktionellen, zur radikalischen Polymerisation befähigten, siliziumhaltigen Komponente(n),
E) gegebenenfalls einer oder mehreren mindestens monofunktionellen, zur radikalischen Polymerisation befähigten, fluorhaltigen Komponente(n),
F) einem oder mehreren einfach oder mehrfach olefinisch ungesättigten, gegebenenfalls vernetzenden, Makromonomeren, die jeweils mindestens ein Sauerstoff-, Stickstoff-, Schwefel- oder Phosphoratom besitzen und ein zahlenmittleres Molekulargewicht größer oder gleich 200 g/mol aufweisen, wobei es sich bei den Makromonomeren nicht um eine siliziumhaltige Komponente D) oder fluorhaltige Komponente E) handelt, und wobei es sich bei den Makromonomeren F) um Verbindungen der Formel (IV) handelt, 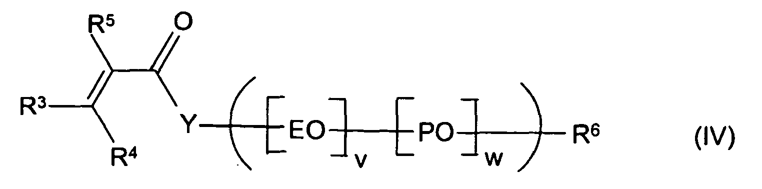 worin
R₃ und R₄ gleich H oder -CH₃ sind,
R₅ gleich H oder -CH₃ ist;
R₆ gleich einem n-aliphatischen, iso-aliphatischen, olefinischen, cycloaliphatischen, arylaliphatischen oder aromatischen (C₁-C₃₀)-Kohlenwasserstoffrest ist,
EO eine Ethylenoxideinheit und
PO eine Propylenoxideinheit ist,
v und w unabhängig voneinander 0 bis 500 betragen, wobei die Summe aus v und w im Mittel ≥ 1 sein muss und die Verteilung der EO- und PO-Einheiten über die Makromonomerkette statistisch, blockartig, alternierend oder gradientenartig sein kann und
Y -O-, -C(O)-, -C(O)-O-, -S-, -O-CH₂-CH(O-)-CH₂OH,
-O-CH₂-CH(OH)-CH₂O-, -O-SO₂-O-, -O-SO-O-, -PH-, -P(CH₃)-, -PO₃-, -NH-oder -N(CH₃)- ist, und
G) wobei die Copolymerisation gegebenenfalls in Gegenwart mindestens eines polymeren Additivs mit zahlenmittleren Molekulargewichten von 200 g/mol bis 10⁹ g/mol erfolgt,
enthalten.

2. Haarbehandlungsmittel nach Anspruch 1, **dadurch gekennzeichnet, dass** es sich bei den Comonomeren B) um ungesättigte Carbonsäuren, Salze ungesättigter Carbonsäuren, Anhydride ungesättigter Carbonsäuren, Ester ungesättigter Carbonsäuren mit aliphatischen, olefinischen, cycloaliphatischen, arylaliphatischen oder aromatischen Alkoholen mit 1 bis 22 C-Atomen, offenkettige N-Vinylamide, cyclische N-Vinylamide mit einer Ringgröße von 3 bis 9, Amide der Acrylsäure, Amide der Methacrylsäure, Amide substituierter Acrylsäuren, Amide substituierter Methacrylsäuren, 2-Vinylpyridin, 4-Vinylpyridin, Vinylacetat; Styrol, Acrylnitril, Vinylchlorid, Vinylidenchlorid, Tetrafluorethylen, Vinylphosphonsäure oder deren Ester oder Salze, Vinylsulfonsäure oder deren Ester oder Salze, Allylphosphonsäure oder deren Ester oder Salze und/oder Methallylsulfonsäure oder deren Ester oder Salze handelt.

3. Haarbehandlungsmittel nach Anspruch 1 und/oder 2, **dadurch gekennzeichnet, dass** es sich bei den Comonomeren C) um Diallyldimethylammoniumchlorid (DADMAC),
[2-(Methacryloyloxy)ethyl]trimethylammoniumchlorid (MAPTAC),
[2-(Acryloyloxy)ethyl]trimethylammoniumchlorid,
[2-Methacrylamidoethyl]trimethylammoniumchlorid,
[2-(Acrylamido)ethyl]trimethylammoniumchlorid,
N-Methyl-2-vinylpyridiniumchlorid
N-Methyl-4-vinylpyridiniumchlorid
Dimethylaminoethylmethacrylat,
Dimethylaminopropylmethacrylamid,
Methacryloylethyl-N-oxid und/oder
Methacryloylethyl-betain handelt.

4. Haarbehandlungsmittel nach mindestens einem der Ansprüche 1 bis 3, **dadurch gekennzeichnet, dass** es sich bei den siliziumhaltigen Komponenten D) um Verbindungen der Formel (I)
R¹ - Z- [(Si(R³R⁴)-O-)_{w}-(Si(R⁵R⁶)-O)ₓ-]- R² (I)
handelt, wobei
R¹ einen Vinyl-, Allyl-, Methallyl-, Methylvinyl-, Acryl-, Methacryl-, Crotonyl-, Senecionyl-, Itaconyl-, Maleinyl-, Fumaryl- oder ein Styrylrest darstellt;
Z eine chemische Brücke, bevorzugt ausgewählt aus -O-, -((C₁-C₅₀) Alkyl)-, -((C₆-C₃₀) Aromat)-, -((C₅-C₈) Cycloalkyl)-, -((C₁-C₅₀) Alkenyl)-, -(Polypropylenoxid)ₙ-, -(Polyethylenoxid)ₒ-, -(Polypropylenoxid)ₙ(Polyethylenoxid)ₒ-, wobei n und o unabhängig voneinander Zahlen von 0 bis 200 bedeuten und die Verteilung der EO/PO-Einheiten statistisch oder blockförmig sein kann; -((C₁-C₁₀) Alkyl)-(Si(OCH₃)₂)- und -(Si(OCH₃)₂)-, darstellt;
R³ R⁴, R⁵ und R⁶ unabhängig voneinander -CH₃, -O-CH₃, -C₆H₅ oder -O-C₆H₅ bedeuten;
w, x Zahlen von 0 bis 500 bedeuten, wobei entweder w oder x größer Null sein muss, und
R² einen gesättigten oder ungesättigten, aliphatischen cycloaliphatischen, arylaliphatischen oder aromatischen Rest mit jeweils 1 bis 50 C-Atomen oder eine Gruppe der Formeln -OH, -NH₂, -N(CH₃)₂, -R⁷ oder eine Gruppe -Z-R¹ bedeutet, wobei Z und R¹ die oben genannten Bedeutungen haben und
R⁷ eine Gruppe der Formel -O-Si(CH₃)₃, -O-Si(Phenyl)₃, -O-Si(O-Si(CH₃)₃)₂CH₃) und -O-Si(O-Si(Ph)₃)₂Ph) bedeutet.

5. Haarbehandlungsmittel nach mindestens einem der Ansprüche 1 bis 4, **dadurch gekennzeichnet, dass** es sich bei den fluorhaltigen Komponenten E) um Verbindungen der Formel (II)
R¹-Y-CᵣH₂ᵣCₛF₂ₛCF₃ (II)
handelt, wobei
R¹ eine polymerisationsfähige Funktion aus der Gruppe der vinylisch ungesättigten Verbindungen, bevorzugt einen Vinyl-, Allyl-, Methallyl-, Methylvinyl-, Acryl-, Methacryl-, Crotonyl-, Senecionyl-, Itaconyl-, Maleinyl-, Fumaryl- oder Styrylrest, darstellt;
Y eine chemische Brücke, bevorzugt -O-, -C(O)-, -C(O)-O-, -S-, -O-CH₂-CH(O-)-CH₂OH, -O-CH₂-CH(OH)-CH₂-O-, -O-SO₂-O-, -O-S(O)-O-, -PH-, -P(CH₃)-, -PO₃-, -NH-, -N(CH₃)-, -O-(C₁-C₅₀)Alkyl-O-, -O-Phenyl-O-, -O-Benzyl-O-, -O-(C₅-C₈)Cycloalkyl-O-, -O-(C₁-C₅₀)Alkenyl-O-, -O-(CH(CH₃)-CH₂-O)ₙ-, -O-(CH₂-CH₂-O)ₙ- und -O-([CH-CH2-O]ₙ-[CH₂-CH₂-O]ₘ)ₒ-, wobei n, m und o unabhängig voneinander Zahlen von 0 bis 200 bedeuten, darstellt und
r, s stöchiometrische Koeffizienten darstellen, die unabhängig voneinander Zahlen zwischen 0 und 200 sein können.

6. Haarbehandlungsmittel nach mindestens einem der Ansprüche 1 bis 5, **dadurch gekennzeichnet, dass** es sich bei den polymeren Additiven G) um Homo-oder Copolymere aus N-Vinylformamid, N-Vinylacetamid, N-Vinylpyrrolidon, Ethylenoxid, Propylenoxid, Acryloyldimethyltaurinsäure, N-Vinylcaprolacton, N-Vinylmethylacetamid, Acrylamid, Acrylsäure, Methacrylsäure, N-Vinylmorpholid, Hydroxymethylmethacrylat, Diallyldimethylammoniumchlorid (DADMAC) und/oder [2-(Methacryloyloxy)ethyl]trimethylammoniumchlorid (MAPTAC); Polyalkylenglykole und/oder Alkylpolyglykole handelt.

7. Haarbehandlungsmittel nach mindestens einem der Ansprüche 1 bis 6, **dadurch gekennzeichnet, dass** die Copolymerisation in Gegenwart mindestens eines polymeren Additivs G) erfolgt.

8. Haarbehandlungsmittel nach mindestens einem der Ansprüche 1 bis 7, **dadurch gekennzeichnet, dass** die Copolymere vernetzt sind.

9. Haarbehandlungsmittel nach mindestens einem der Ansprüche 1 bis 8, **dadurch gekennzeichnet, dass** die Copolymere durch Fällungspolymerisation in tert.-Butanol hergestellt werden.

10. Haarbehandlungsmittel nach mindestens einem der Ansprüche 1 bis 9, **dadurch gekennzeichnet, dass** die Copolymere wasserlöslich oder wasserquellbar sind.

11. Haarbehandlungsmittel nach mindestens einem der Ansprüche 1 bis 10, **dadurch gekennzeichnet, dass** sie, bezogen auf die fertigen Mittel, 0,01 bis 10 Gew.-% der Copolymere enthalten.

12. Haarbehandlungsmittel nach mindestens einem der Ansprüche 1 bis 11, **dadurch gekennzeichnet, dass** sie Ölkörper, Emulgatoren, Co-Emulgatoren, kationische Polymere, Filmbildner, Überfettungsmittel, feuchtigkeitsspendende Mittel, Stabilisatoren, biogene Wirkstoffe, Glycerin, Konservierungsmittel, Perlglanzmittel, Farb- und Duftstoffe, Lösungsmittel, Hydrotrope, Trübungsmittel, Verdickungsmittel, Dispergiermittel, Eiweißderivate, Polypeptide auf natürlicher und synthetischer Basis, Eigelb, Lecithin, Lanolin, Lanolinderivate, Fettalkohole, Silicone, Stoffe mit keratolytischer und keratoplastischer Wirkung, Enzyme, Trägersubstanzen, Antioxidation, Lichtschutzstoffe UV-Lichtschutzfilter, biogene Wirkstoffe, pharmazeutisch aktive Wirkstoffe und/oder Antischuppenmittel enthalten.

13. Haarbehandlungsmittel nach mindestens einem der Ansprüche 1 bis 12 **dadurch gekennzeichnet, dass** es sich dabei um Spülungen, Kuren, Sprühkuren Lotionen, Cremes, Gele, Schäume und Sprays handelt.

## Claims

1. A cosmetic or dermatological hair treatment composition which comprises at least one copolymer obtainable by free-radical copolymerization of
A) acryloyldimethyltaurine and/or acryloyldimethyltaurates,
B) if desired, one or more further olefinically unsaturated, noncationic, optionally crosslinking comonomers which have at least one oxygen, nitrogen, sulfur or phosphorus atom and possess a molecular weight of less than 500 g/mol,
C) one or more olefinically unsaturated, cationic comonomers which have at least one oxygen, nitrogen, sulfur or phosphorus atom and possess a molecular weight of less than 500 g/mol,
D) if desired, one or more silicon-containing components capable of free-radical polymerization and having a functionality of at least one,
E) if desired, one or more fluorine-containing components capable of free-radical polymerization and having a functionality of at least one,
F) one or more olefinically mono- or polyunsaturated, optionally crosslinking macromonomers each possessing at least one oxygen, nitrogen, sulfur or phosphorus atom and having a number-average molecular weight of greater than or equal to 200 g/mol, the macromonomers not being a silicon-containing component D) or fluorine-containing component E), and where the macromonomers F) are compounds of the formula (IV) 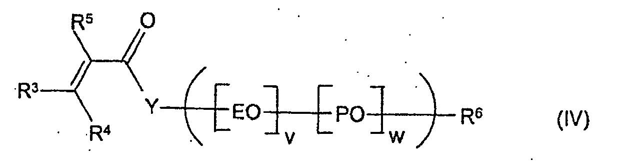 in which
R³ and R⁴ are H or -CH₃,
R⁵ is H or -CH₃ ;
R⁶ is an n-aliphatic, iso-aliphatic, olefinic, cycloaliphatic, arylaliphatic or aromatic (C₁-C₃₀) hydrocarbon radical,
EO is an ethylene oxide unit and
PO is a propylene oxide unit,
v and w amount independently of one another to from 0 to 500, it being necessary for the sum of v and w to be on average ≥ 1 and it being possible for the distribution of the EO and PO units over the macromonomer chain to be random, blocklike, alternating or gradient like and
Y is -O-, -C (O)-, -C(O)-O-, -S-, -O-CH₂-CH (O-) -CH₂OH, -O-CH₂-CH (OH) -CH₂O-, -O-SO₂-O-, -O-SO-O-, -PH-, -P(CH₃) -, -PO₃-, -NH-, or -N(CH₃)-, and
G) the copolymerization taking place if desired in the presence of at least one polymeric additive having number-average molecular weights of from 200 g/mol to 10⁹ g/mol.

2. A hair treatment composition as claimed in claim 1, wherein the comonomers B) are unsaturated carboxylic acids, salts of unsaturated carboxylic acids, anhydrides of unsaturated carboxylic acids, esters of unsaturated carboxylic acids with aliphatic, olefinic, cycloaliphatic, arylaliphatic or aromatic alcohols having 1 to 22 carbon atoms, open-chain N-vinyl amides, cyclic N-vinyl amides having a ring size of from 3 to 9, amides of acrylic acid, amides of methacrylic acid, amides of substituted acrylic acids, amides of substituted methacrylic acids, 2-vinylpyridine, 4-vinylpyridine, vinyl acetate; styrene, acrylonitrile, vinyl chloride, vinylidene chloride, tetrafluoroethylene, vinylphosphonic acid or the esters or salts thereof, vinylsulfonic acid or the esters or salts thereof, allylphosphonic acid or the esters or salts thereof and/or methallylsulfonic acid or the esters or salts thereof.

3. A hair treatment composition as claimed in claim 1 and/or 2, wherein the comonomers C) are
diallyldimethylammonium chloride (DADMAC),
[2-(methacryloyloxy)ethyl]trimethylammonium chloride (MAPTAC),
[2-(acryloyloxy)ethyl]trimethylammonium chloride,
[2-methacrylamidoethyl]trimethylammonium chloride,
[2-(acrylamido)ethyl]trimethylammonium chloride,
N-methyl-2-vinylpyridinium chloride,
N-methyl-4-vinylpyridinium chloride,
dimethylaminoethyl methacrylate,
dimethylaminopropylmethacrylamide,
methacryloylethyl N-oxide and/or
methacryloylethylbetaine.

4. A hair treatment composition as claimed in at least one of claims 1 to 3, wherein the silicon-containing components D) are compounds of the formula (I)
R¹ - Z- [(Si(R³R⁴)-O-)_{w}-(Si(R⁵R⁶)-O)ₓ-]- R² (I)
where
R¹ represents a vinyl, allyl, methallyl, methylvinyl, acryloyl, methacryloyl, crotonyl, senecionyl, itaconyl, maleyl, fumaryl or styryl radical;
Z is a chemical bridge, preferably selected from -O-- ((C₁-C₅₀) alkyl) -, - ((C₆-C₃₀) aromatic) -, - ((C₅-C₈) cycloalkyl) -, - ((C₁-C₅₀) alkenyl) -, -(polypropylene oxide)ₙ-, (polyethylene oxide)ₒ-, -(polypropylene oxide)ₙ(polyethylene oxide)ₒ-, where n and o independently of one another denote numbers from 0 to 200, and the distribution of the EO/PO units can be random or in the form of blocks;
-((C1-C₁₀)alkyl)-(Si(OCH₃)₂)- and - (Si (OCH₃)₂)-;
R³, R⁴, R⁵, and R⁶ independently of one another are -CH₃, -O-CH₃, -C₆H₅ or -O-C₆H₅;
w, x denote numbers from 0 to 500, it being necessary for either w or x to be greater than zero; and
R² is a saturated or unsaturated aliphatic, cycloaliphatic, arylaliphatic or aromatic radical having in each case 1 to 50 carbon atoms or a group of the formulae -OH, -NH₂, -N(CH₃)₂ , -R⁷ or a group -Z-R¹, where Z and R¹ have the meanings mentioned above, and
R⁷ is a group of the formula -O-Si (CH₃)₃, -O-Si(phenyl)₃,
-O-Si(O-Si(CH₃)₃)₂CH₃) and -O-Si(O-Si(Ph)₃)₂Ph).

5. A hair treatment composition as claimed in at least one of claims 1 to 4, wherein the fluorine-containing components E) are compounds of the formula (II)
R¹-Y-CᵣH₂ᵣCₛF₂ₛCF₃ (II)
where
R¹ represents a polymerizable function from the group of the vinylically unsaturated compounds, preferably a vinyl, allyl, methallyl, methylvinyl, acryloyl, methacryloyl, crotonyl, senecionyl, itaconyl, maleyl, fumaryl or styryl radical;
Y is a chemical bridge, preferably -O-, -C(O)-,-C(O)-O-, -S-,
-O-CH₂-CH(O-)-CH₂OH, -O-CH₂-CH(OH)-CH₂-O-, -O-SO₂-O-,
-O-S(O)-O-, -PH-, -P(CH₃)-, -PO₃-, -NH-, -N(CH₃)-, -O- (C₁-C₅₀) alkyl-O-,
-O-phenyl-O-, -O-benzyl-O-, -O-(C₅-C₈)cycloalkyl-O-,
-O- (C₁-C₅₀)alkenyl-O-, -O-(CH(CH₃) =CH₂-O)ₙ-, -O-(CH₂-CH₂-O)ₙ-, and
-O-([CH-CH₂-O]ₙ-[CH₂-CH₂-O]ₘ)ₒ-, where n, m, and o independently of one another denote numbers from 0 to 200, and
r, s are stoichiometric coefficients which independently of one another can be numbers between 0 and 200.

6. A hair treatment composition as claimed in at least one of claims 1 to 5, wherein the polymeric additives G) are homopolymers or copolymers of N-vinylformamide, N-vinylacetamide, N-vinylpyrrolidone, ethylene oxide, propylene oxide, acryloyldimethyltaurine, N-vinylcaprolactone, N-vinylmethylacetamide, acrylamide, acrylic acid, methacrylic acid, N-vinylmorpholide, hydroxymethyl methacrylate, diallyldimethylammonium chloride (DADMAC) and/or [2-(methacryloyloxy)ethyl]trimethylammonium chloride (MAPTAC); polyalkylene glycols and/or alkylpolyglycols.

7. A hair treatment composition as claimed in at least one of claims 1 to 6, wherein the copolymerization takes place in the presence of at least one polymeric additive G).

8. A hair treatment composition as claimed in at least one of claims 1 to 7, wherein the copolymers are crosslinked.

9. A hair treatment composition as claimed in at least one of claims 1 to 8, wherein the copolymers are prepared by precipitation polymerization in tert-butanol.

10. A hair treatment composition as claimed in at least one of claims 1 to 9, wherein the copolymers are water-soluble or water-swellable.

11. A hair treatment composition as claimed in at least one of claims 1 to 10, which comprises, based on the finished composition, from 0.01 to 10% by weight of the copolymers.

12. A hair treatment composition as claimed in at least one of claims 1 to 11, comprising oily substances, emulsifiers, coemulsifiers, cationic polymers, film formers, superfatting agents, moisturizers, stabilizers, active biogenic substances, glycerol, preservatives, pearlizing agents, dyes and fragrances, solvents, hydrotropic agents, opacifiers, thickeners, dispersants, protein derivatives, polypeptides on a natural or synthetic basis, egg yolk, lecithin, lanolin, lanolin derivatives, fatty alcohols, silicones, substances having a keratolytic and keratoplastic effect, enzymes, carrier substances, antioxidants, light stabilizers, UV light protection filters, active pharmaceutical substances and/or antidandruff agents.

13. A hair treatment composition as claimed in at least one of claims 1 to 12, which is a rinse, cure, spray cure, lotion, cream, gel, foam or spray.

## Revendications

1. Agents cosmétiques et dermatologiques de traitement des cheveux, **caractérisés en ce qu'**ils contiennent au moins un copolymère, pouvant être obtenu par copolymérisation radicalaire
A) d'acide acryloyldiméthyltaurique et/ou d'acryloyldiméthyltaurates,
B) le cas échéant d'un ou de plusieurs autres comonomères oléfiniquement insaturés, non cationiques, le cas échéant réticulants, qui présentent au moins un atome d'oxygène, d'azote, de soufre ou de phosphore et qui présentent un poids moléculaire inférieur à 500 g/mole,
C) d'un ou de plusieurs comonomères oléfiniquement insaturés, cationiques, qui présentent au moins un atome d'oxygène, d'azote, de soufre ou de phosphore et qui présentent un poids moléculaire inférieur à 500 g/mole,
D) le cas échéant d'un ou de plusieurs composant(s) silicié(s), au moins monofonctionnel (s) , apte(s) à une polymérisation par voie radicalaire,
E) le cas échéant d'un ou de plusieurs composant(s) fluoré(s), au moins monofonctionnel (s), apte(s) à une polymérisation par voie radicalaire,
F) d'un ou de plusieurs macromonomères oléfiniquement monoinsaturés ou polyinsaturés, le cas échéant réticulants, qui présentent à chaque fois au moins un atome d'oxygène, d'azote, de soufre ou de phosphore et qui présentent un poids moléculaire numérique moyen supérieur ou égal à 200 g/mole, où il ne s'agit pas, pour les macromonomères, d'un composant silicié D) ou d'un composant fluoré E) et où il s'agit pour les macromonomères F) de composants de formule (IV), 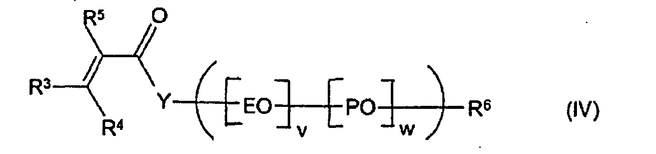 où
R³ et R⁴ représentent H ou -CH₃,
R⁵ représente H ou -CH₃ ;
R⁶ représente un radical hydrocarboné en C₁-C₃₀ n-aliphatique, isoaliphatique, oléfinique, cycloaliphatique, arylaliphatique ou aromatique,
EO représente une unité d'oxyde d'éthylène et
PO représente une unité d'oxyde de propylène,
v et w valent, indépendamment l'un de l'autre, 0 à 500, où la somme de v et de w doit en moyenne être ≥ 1 et la répartition des unités EO et PO sur la chaîne de macromonomère peut être statistique, en blocs, en alternance ou en gradient et
Y représente -O-, -C(O)-, -C(O)-O-, -S-, -O-CH₂-CH(O-)-CH₂OH, -O-CH₂-CH(OH)-CH₂O-, -O-S0₂-O-, -O-SO-O-, -PH-, -P(CH₃)-, -PO₃-, -NH- ou -N(CH₃)-, et
G) où la copolymérisation est le cas échéant réalisée en présence d'au moins un additif polymère présentant des poids moléculaires numériques moyens de 200 g/mole à 10⁹ g/mole.

2. Agents de traitement des cheveux selon la revendication 1, **caractérisés en ce qu'**il s'agit, pour les comonomères B), d'acides carboxyliques insaturés, de sels d'acides carboxyliques insaturés, d'anhydrides d'acides carboxyliques insaturés, d'esters d'acides carboxyliques insaturés avec des alcools aliphatiques, oléfiniques, cycloaliphatiques, arylaliphatiques ou aromatiques comprenant 1 à 22 atomes de carbone, de N-vinylamides à chaîne ouverte, de N-vinylamides cycliques présentant une taille de cycle de 3 à 9, d'amides de l'acide acrylique, d'amides de l'acide méthacrylique, d'amides d'acides acryliques substitués, d'amides d'acides méthacryliques substitués, de 2-vinylpyridine, de 4-vinylpyridine, d'acétate de vinyle, de styrène, d'acrylonitrile, de chlorure de vinyle, de chlorure de vinylidène, de tétrafluoroéthylène, d'acide vinylphosphonique ou de ses esters ou sels, d'acide vinylsulfonique ou de ses esters ou sels, d'acide allylphosphonique ou de ses esters ou sels et/ou d'acide méthallylsulfonique ou de ses esters ou sels.

3. Agents de traitement des cheveux selon la revendication 1 et/ou 2, **caractérisés en ce qu'**il s'agit, pour les comonomères C), de chlorure de diallyldiméthylammonium (DADMAC), de chlorure de [2-(méthacryloyloxy)éthyl]triméthylammonium (MAPTAC), de chlorure de [2-(acryloyloxy)éthyl]triméthylammonium, de chlorure de [2-méthacrylamidoéthyl]triméthylammonium, de chlorure de [2-(acrylamido)éthyl]triméthylammonium, de chlorure de N-méthyl-2-vinylpyridinium, de chlorure de N-méthyl-4-vinylpyridinium, de méthacrylate de diméthylaminoéthyle, de diméthylaminopropylméthacrylamide, de méthacryloyléthyl-N-oxyde et/ou de méthacryloyléthylbétaïne.

4. Agents de traitement des cheveux selon au moins l'une quelconque des revendications 1 à 3, **caractérisés en ce qu'**il s'agit, pour les composants siliciés D) de composés de formule (I)
R¹-Z- [ (Si (R³R⁴) -O-)_{w}- (Si(R⁵R⁶)-O)ₓ-] -R² (I)
où
R¹ représente un radical vinyle, allyle, méthallyle, méthylvinyle, acryle, méthacryle, crotonyle, sénécionyle, itaconyle, maléinyle, fumaryle ou styryle ;
Z représente un pont chimique, de préférence choisi parmi -O-, - ((C₁-C₅₀) alkyl) -, -((C₆-C₃₀)aromatique)-, -((C₅-C₈)cycloalkyl)-, - ((C₁-C₅₀)alcényl)-, -(poly(oxyde de propylène))ₙ-, - (poly (oxyde d'éthylène))ₒ-, - (poly (oxyde de propylène))ₙ(poly (oxyde d'éthylène))ₒ-, où n et o signifient, indépendamment l'un de l'autre, des nombres de 0 à 200 et la répartition des unités EO/PO peut être statistique ou en forme de blocs ; -((C₁-C₁₀)alkyl)-(Si(OCH₃)₂)- et -(Si(OCH₃)₂)- ;
R³, R⁴, R⁵ et R⁶ signifient, indépendamment les uns des autres, -CH₃, -O-CH₃, -C₆H₅ ou -O-C₆H₅;
w, x signifient des nombres de 0 à 500, où soit w, soit x doit être supérieur à zéro, et
R² signifie un radical saturé ou insaturé, aliphatique, cycloaliphatique, arylaliphatique ou aromatique, comprenant à chaque fois 1 à 50 atomes de carbone ou un groupe de formule -OH, -NH₂, -N(CH₃)₂, -R⁷ ou un groupe -Z-R¹, où Z et R¹ ont les significations susmentionnées et
R⁷ signifie un groupe de formule -O-Si (CH₃)₃, -0-Si(phényle)₃, -O-Si(O-Si(CH₃)₃)₂CH₃) et -O-Si(O-Si(Ph)₃)₂Ph).

5. Agents de traitement des cheveux selon au moins l'une quelconque des revendications 1 à 4, **caractérisés en ce qu'**il s'agit, pour les composants fluorés E), de composés de formule (II)
R¹-Y-CᵣH₂ᵣCₛF₂ₛCF₃ (II)
où
R¹ représente une fonction apte à la polymérisation du groupe des composés vinyliquement insaturés, de préférence un radical vinyle, allyle, méthallyle, méthylvinyle, acryle, méthacryle, crotonyle, sénécionyle, itaconyle, maléinyle, fumaryle ou styryle ;
Y représente un pont chimique, de préférence -O-, -C (O) -, -C(O)-O-, -S-, -O-CH₂-CH(O-)-CH₂OH, -O-CH₂-CH(OH)-CH₂-O-, -O-SO₂-O- ,-O-S(O)-O-, -PH-, -P(CH₃)-, -PO₃-, -NH-, -N(CH₃)-, -O-(C₁-C₅₀)alkyl-O-, -O-phényl-O-, -O-benzyl-O-, -O-(C₅-C₈) cycloalkyl-O-, -O- (C₁-C₅₀) alcényl-O-, -O-(CH(CH₃)-CH₂-O)ₙ-, -O-(CH₂-CH₂-O)ₙ- et -O-([CH-CH₂-O]ₙ-[CH₂-CH₂-O]ₘ)ₒ-, où n, m et o signifient, indépendamment les uns des autres, des nombres de 0 à 200, et
r, s représentent des coefficients stoechiométriques, qui peuvent représenter, indépendamment l'un de l'autre, des nombres entre 0 et 200.

6. Agents de traitement des cheveux selon au moins l'une quelconque des revendications 1 à 5, **caractérisés en ce qu'**il s'agit pour les additifs polymères G) d'homopolymères ou de copolymères de N-vinylformamide, de N-vinylacétamide, de N-vinylpyrrolidone, d'oxyde d'éthylène, d'oxyde de propylène, d'acide acryloyldiméthyltaurique, de N-vinylcaprolactone, de N-vinylméthylacétamide, d'acrylamide, d'acide acrylique, d'acide méthacrylique, de N-vinylmorpholide, de méthacrylate d'hydroxyméthyle, de chlorure de diallyldiméthylammonium (DADMAC) et/ou de chlorure de [2-(méthacryloyloxy)éthyl]triméthylammonium (MAPTAC) ; de polyalkylèneglycols et/ou d'alkylpolyglycols.

7. Agents de traitement des cheveux selon au moins l'une quelconque des revendications 1 à 6, **caractérisés en ce que** la copolymérisation est réalisée en présence d'au moins un additif polymère G).

8. Agents de traitement des cheveux selon au moins l'une quelconque des revendications 1 à 7, **caractérisés en ce que** les copolymères sont réticulés.

9. Agents de traitement des cheveux selon au moins l'une quelconque des revendications 1 à 8, **caractérisés en ce que** les copolymères sont préparés par une polymérisation par précipitation dans du tert-butanol.

10. Agents de traitement des cheveux selon au moins l'une quelconque des revendications 1 à 9, **caractérisés en ce que** les copolymères sont solubles ou gonflables dans l'eau.

11. Agents de traitement des cheveux selon au moins l'une quelconque des revendications 1 à 10, **caractérisés en ce qu'**ils contiennent, par rapport aux agents finis, 0,01 à 10% en poids des copolymères.

12. Agents de traitement des cheveux selon au moins l'une quelconque des revendications 1 à 11, **caractérisés en ce qu'**ils contiennent des corps huileux, des émulsifiants, des co-émulsifiants, des polymères cationiques, des agents filmogènes, des agents de relipidation, des agents d'hydratation, des stabilisateurs, des agents actifs biogènes, du glycérol, des conservateurs, des agents nacrés, des colorants et des parfums, des solvants, des agents hydrotropes, des agents conférant un trouble, des épaississants, des dispersants, des dérivés protéiniques, des polypeptides à base naturelle et synthétique, du jaune d'oeuf, de la lécithine, de la lanoline, des dérivés de lanoline, des alcools gras, des silicones, des substances avec un effet kératolytique et kératoplastique, des enzymes, des substances support, des antioxydants, des agents de protection contre la lumière, des filtres de protection contre la lumière UV, des substances pharmaceutiquement actives et/ou des agents antipelliculaires.

13. Agents de traitement des cheveux selon au moins l'une quelconque des revendications 1 à 12, **caractérisés en ce qu'**il s'agit d'après-shampooings, de cures, de cures pulvérisées, de lotions, de crèmes, de gels, de mousses et de sprays.
